# EUROPEAN PATENT SPECIFICATION

(11) **EP 3 960 154 B1**
(45) Date of publication and mention of the grant of the patent: **26.11.2025**
(21) Application number: 20794952.0
(22) Date of filing: 17.04.2020
(51) Int. Cl.: A61K 8/98, A61K 8/9789, A61Q 19/00, A61Q 19/08, A61Q 19/02, A61K 35/50, A61K 36/73, A61P 17/00, A23L 33/10, A23L 33/105, A61K 9/00, A61K 8/14, A61K 8/99, C12N 5/073, C12N 5/079, C12N 5/0797

(54) **FUNCTIONAL COMPOSITION CONTAINING IMMORTALIZED STEM CELL-DERIVED EXOSOME-RICH CULTURE MEDIUM AND ROSEBUD EXTRACT AS ACTIVE INGREDIENTS**
FUNKTIONELLE ZUSAMMENSETZUNG MIT AUS IMMORTALISIERTEN STAMMZELLEN ABGELEITETEM EXOSOMENREICHEM KULTURMEDIUM UND ROSENBLÜTENEXTRAKT ALS WIRKSTOFFE
COMPOSITION FONCTIONNELLE CONTENANT UN MILIEU DE CULTURE RICHE EN EXOSOMES DÉRIVÉ D'UNE CELLULE SOUCHE IMMORTALISÉE ET UN EXTRAIT DE BOURGEON DE ROSE EN TANT QUE PRINCIPES ACTIFS

(30) Priority: 26.04.2019 KR 20190049134
(43) Date of publication of application: 02.03.2022
(73) Proprietor: Chungbuk National University Industry-Academic Cooperation Foundation, Cheongju-si, Chungcheongbuk-do 28644 (KR); Designed Cells Co., Ltd., Cheongju-si, Chungcheongbuk-do 28576 (KR)
(72) Inventor: KIM, Yun Bae, Sejong-si 30100 (KR); JEONG, Heon Sang, Cheongju-si Chungcheongbuk-do 28114 (KR); CHOI, Ehn Kyoung, Jeonju-si Jeollabuk-do 54904 (KR)
(74) Representative: Eisenführ Speiser
(86) International application number: PCT/KR2020/005170
(87) International publication number: WO 2020/218781

(56) References cited:
- KR-A- 20130 109 999
- KR-A- 20130 109 999
- KR-A- 20160 022 119
- KR-A- 20160 022 119
- KR-A- 20160 061 945
- KR-A- 20160 061 945
- KR-A- 20180 131 158
- KR-A- 20180 131 158
- JANG YOUNG-EUN: "Mamonde "Water your hot summer skin"", EDAILY NEWS, 7 June 2012 (2012-06-07), XP055752851, Retrieved from the Internet <URL:https://www.edaily.co.kr/news/read?newsId=02299286599559424&mediaCodeNo=257> [retrieved on 20201123]
- JEON JEONG HEE ET AL: "Anti-allergic effects of white rose petal extract and anti-atopic properties of its hexane fraction", vol. 32, no. 6, 1 June 2009 (2009-06-01), KR, pages 823 - 830, XP093004648, ISSN: 0253-6269, Retrieved from the Internet <URL:https://link.springer.com/content/pdf/10.1007/s12272-009-1602-6.pdf?pdf=button> DOI: 10.1007/s12272-009-1602-6
- SHIN EUN JU ET AL: "Extraction conditions forpetal extracts with anti-skin aging activities", FOOD SCIENCE AND BIOTECHNOLOGY, THE KOREA SOC. OF FOOD SCIENCE AND TECHNOLOGY, HEIDELBERG, vol. 28, no. 5, 26 March 2019 (2019-03-26), pages 1439 - 1446, XP036913839, ISSN: 1226-7708, [retrieved on 20190326], DOI: 10.1007/S10068-019-00596-7
- HAN CHAO ET AL: "Exosomes and Their Therapeutic Potentials of Stem Cells", vol. 2016, 6 December 2015 (2015-12-06), US, pages 1 - 11, XP055889604, ISSN: 1687-966X, Retrieved from the Internet <URL:https://downloads.hindawi.com/journals/sci/2016/7653489.pdf> DOI: 10.1155/2016/7653489
- JANG YOUNG-EUN: "Mamonde "Water your hot summer skin"", EDAILY NEWS, 7 June 2012 (2012-06-07), XP055752851, Retrieved from the Internet <URL:https://www.edaily.co.kr/news/read?newsId=02299286599559424&mediaCodeNo=257>

## Description

### [Technical Field]

The present disclosure relates to a composition containing an immortalized stem cell-derived exosome-rich conditioned medium and a rosebud extract as active ingredients as well as to a method for preparing said composition and a composition for use in a method for preventing or treating atopic dermatitis or pigmentation diseases.

### [Background Art]

As the living environment of city dwellers has recently changed, they are increasingly exposed to various allergens that cause immune hypersensitivity, such as mites living on carpets at home, dry climate, fine dust due to industrialization and environmental pollution, pet hair, pollen, food additives, etc. Among them, atopic dermatitis occurs at a rate of 10 to 20% in infants and 1 to 3% in adults, and its prevalence is steadily increasing in recent years. For modern people who pursue well-being, skin anti-wrinkle, moisturizing, and whitening can achieve significant effects by using various functional cosmetics that can block UV rays and prevent aging in terms of skin beauty. However, atopic dermatitis is a serious disease that causes severe dermatitis and itchiness as well as psychological stress due to the continuous action of antigens. It is very difficult to treat and requires a long time for treatment and improvement.

In addition, an allergic reaction is an excessive immune response of our body, which is one of four types mechanically. Atopy, known as type 1 hypersensitivity reaction, is characterized by excessive activation of mast cells and the like by immunoglobulin E (IgE). In the early stage, allergens that induce atopic hypersensitivity reaction activate type 2 helper T cells (Th2 cells). It then reacts with B-cells that produce antibodies containing immunoglobulin E. When an allergen reacts with immunoglobulin E cross-linked to Fcε receptor I (FcεRI) on the surface of mast cells, histamine is released from the mast cells, causing an immediate hypersensitivity reaction, that is, anaphylactic shock reaction. In atopic dermatitis, Th2 cells produce a large amount of IL-4, which stimulates plasma cells to produce immunoglobulin E. Therefore, as the most effective anti-allergic and anti-atopic candidate substance, it can be a substance that not only inhibits histamine release from mast cells, but also blocks the activation of Th cells, including early Th2 cells and late Th1 cells.

It has recently been found that stem cells improve atopic dermatitis through an immunomodulatory mechanism. For example, it has been found that subcutaneous injection of umbilical cord blood stem cells (UCBSC) can prevent long-term recurrence of atopic dermatitis, and intravascular injection of neural stem cells (NSC) alleviates multiple sclerosis, an autoimmune disease, by controlling the peripheral immune system. These results suggest that stem cells can alleviate hypersensitivity reactions and autoimmune diseases through immunomodulatory functions. Accordingly, stem cells are expected to prevent and treat atopic dermatitis involving type 1 hypersensitivity reaction and associated with Th2 cells.

Furthermore, stem cells or stem cells conditioned medium have been known to have antioxidant, skin regeneration and wound healing effects, and in particular, adipose-derived stem cells (ADSC) promote collagen production and inhibit decomposition to reduce skin wrinkles. Recently, it has been known for its skin whitening effect and been attracting attention as a new material for skin beauty.

However, the anti-wrinkle and whitening effects of stem cells are the results obtained by subcutaneously injecting the stem cells themselves, and there is no research result confirming the efficacy by applying a stem cells conditioned medium (CM) to the skin. This is because the active ingredient in the stem cells conditioned medium is very small in amount and it is mainly composed of protein, so it does not penetrate the skin well. Accordingly, it is necessary to develop a technology capable of maximizing the useful efficacy of stem cells as well as enhancing the skin penetration rate improvement effect.

Conventionally known ingredients exhibiting a whitening effect include an ascorbic acid phosphate ester salt, a hydroquinone derivative, a placental extract, a kojic acid, an ellagic acid, and the like, and a cosmetic composition in which these ingredients are compounded is general. In particular, in recent years, whitening cosmetics using placenta extracts have been in the spotlight, but the supply of such placental extracts is limited and its use is limited in combination with ethical issues. Accordingly, interest in the development of effective new whitening ingredients is increasing.

Accordingly, as it is known that polyphenols contained in plants have a whitening effect, cosmetic compositions using the same have been proposed, and research results have also been reported on the whitening effects of flavanones and hydroxyflavones. As such, attention is focused on functional cosmetics derived from natural plants that are less irritating to the skin and are eco-friendly.

Rose oils are widely used raw materials for perfumes due to their strong and unique scent. However, the medicinal efficacy of rose oils is unknown.

KR20180131158A relates to a method for promoting the secretion of exosomes from stem cells and a functional cosmetic composition.

KR20130109999A relates to a cosmetic composition for whitening containing the neural stem cell culture fluid or the neural stem cell extract as an active ingredient and a manufacturing method thereof.

The product "O² Holding Skin" by Mamonde is a multi-functional product that has both moisturizing, cooling and whitening functions.

KR20160022119A relates to an immortalized cell line having atopic dermatitis improvement, wrinkle improvement and whitening activity, and uses thereof.

KR20160061945A also relates to an immortalized cell line having atopic dermatitis improvement, wrinkle improvement and whitening activity, and uses thereof.

Jeong Hee Jeon an colleagues described the anti-allergic effects of white rose petal extract and the anti-atopic properties of its hexane fraction (Arch Pharm Res Vol 32, No 6, 823-830, 2009).

Eun Ju Shin an colleagues described extraction conditions for *Rosa gallica* petal extracts with anti-skin aging activities (Food Sei Biotechnol (2019) 28(5):1439-1446).

Chao Han an collegueas comprehensively reviewed exosomes and their therapeutic potentials of stem cells (http://dx.doi.org/10.1155/2016/7653489).

Accordingly, the present inventors were researching to discover materials having excellent activity in atopic dermatitis improvement, whitening or wrinkle improvement. As a result of conducting research on stem cells and rose extract, the present inventor identified that the atopic dermatitis, whitening and wrinkle improvement effects were significantly superior when stem cells were prepared as immortalized stem cells and when the immortalized stem cell-derived exosome-rich conditioned medium and the rosebud extract were used simultaneously, and the completed the present disclosure.

### [Disclosure]

### [Technical Problem]

Accordingly, an object of the present disclosure is to provide a composition containing an immortalized stem cell-derived exosome-rich conditioned medium and a rosebud extract as active ingredients.

Another object of the present disclosure is to provide a method for preparing a composition having activity in atopic dermatitis improvement, skin wrinkle improvement and whitening.

Another object of the present disclosure is to provide a composition for use in a method for preventing or treating pigmentation diseases, in which the pharmaceutical composition contains an immortalized stem cell-derived exosome-rich conditioned medium and a rosebud extract as active ingredients.

### [Technical Solution]

An aspect of the present disclosure is directed to providing a composition, including a functional cosmetic composition, containing an immortalized stem cell-derived exosome-rich conditioned medium and a rosebud extract as active ingredients.

In one embodiment of the present disclosure, the immortalized stem cells may be immortalized amniotic stem cells, immortalized neural stem cells, or immortalized oligodendrocyte progenitor cells.

In one embodiment of the present disclosure, the immortalized amniotic stem cells may be immortalized amniotic stem cells, CBNU-AFSC cell line (Accession No.: KCTC12634BP), the immortalized neural stem cells may be immortalized neural stem cells, CBNU-NSC cell line (Accession No.: KCTC12635BP), and the immortalized oligodendrocyte progenitor cells may be immortalized oligodendrocyte progenitor cells, CBNU-NSC.olig2 cell line (Accession No.: KCTC12636BP).

In one embodiment of the present disclosure, the immortalized stem cell-derived exosome-rich conditioned medium may be obtained by treating with TNF-α and interferon gamma (IFN-y) in a culture medium containing immortalized stem cells, and culturing the same under a low oxygen concentration condition of 1 to 5% for 12 to 72 hours.

In one embodiment of the present disclosure, the TNF-α may be treated at a concentration of 3 to 100 ng/ml, and the interferon gamma (IFN-y) may be treated at a concentration of 1 to 10 U/ml.

In one embodiment of the present disclosure, the immortalized stem cells may be 1 × 10⁴ to 1 × 10⁷ cells.

In one embodiment of the present disclosure, the rosebud extract may be a solvent fraction obtained by extraction and fractionation by adding any one of solvents selected from the group consisting of ethanol, butanol and ethyl acetate to a methanol ultrasonic extract obtained by adding methanol to the dried and pulverized rosebuds and sonicating the same.

In one embodiment of the present disclosure, the methanol may use 80% methanol, and ethanol, butanol or ethyl acetate may use 70% ethanol, 70% butanol or 70% ethyl acetate.

In one embodiment of the present disclosure, the rosebud may be a rosebud of a rose selected from the group consisting of Icewing (*Rosa hybrida* Icewing), Onnuri (*Rosa hybrida* Onnuri), Colorado (*Rosa hybrida* Colorado) and rose vines (*Rosa multiflora* Platyphylla thory).

In one embodiment of the present disclosure, the composition may have activity in atopic dermatitis improvement, skin wrinkle improvement or whitening.

In one embodiment of the present disclosure, the composition may be one or more formulations selected from the group consisting of skin lotion, skin softener, skin toner, astringent, lotion, milk lotion, moisture lotion, nutrition lotion, massage cream, nutrition cream, moisture cream, hand cream, foundation, essence, nutrition essence, pack, soap, cleansing foam, cleansing lotion, cleansing cream, body lotion, body cleanser, face wash, treatment, beauty liquids, beauty packs, ointments, gels, liniments, liquids, patches, and sprays.

In addition, the present disclosure provides a method for preparing a composition having activity in atopic dermatitis improvement, skin wrinkle improvement and whitening, in which the method includes: (1) simultaneously treating TNF-α and interferon gamma (IFN-y) in a culture medium containing immortalized stem cells, and culturing the same under a low oxygen concentration condition of 1 to 5% for 12 to 72 hours to obtain an immortalized stem cell-derived exosome-rich conditioned medium; (2) adding any one of solvents selected from the group consisting of ethanol, butanol and ethyl acetate to a methanol ultrasonic extract obtained by adding methanol to dried and pulverized rosebuds and sonicating the same, followed by extraction and fractionation to obtain a solvent fraction for a methanol extract of rosebuds; and (3) mixing the immortalized stem cell-derived exosome-rich conditioned medium obtained in the process (1) and the solvent fraction for a methanol extract of rosebuds obtained in the process (2).

In one embodiment of the present disclosure, the immortalized stem cells may be immortalized amniotic stem cells, CBNU-AFSC cell line (Accession No.: KCTC12634BP), the immortalized neural stem cells may be immortalized neural stem cells, CBNU-NSC cell line (Accession No.: KCTC12635BP), and the immortalized oligodendrocyte progenitor cells may be immortalized oligodendrocyte progenitor cells, CBNU-NSC.olig2 cell line (Accession No.: KCTC12636BP).

In one embodiment of the present disclosure, in the process (1), TNF-α may be treated at a concentration of 3 to 100 ng/ml, and the interferon gamma (IFN-y) may be treated at a concentration of 1 to 10 U/ml.

In one embodiment of the present disclosure, the solvent fraction for the methanol extract of rosebuds obtained in the process (2) in the immortalized stem cell-derived exosome-rich conditioned medium in the process (3) may be a fraction obtained by using ethanol.

In one embodiment of the present disclosure, the solvent fraction for the methanol extract of rosebuds in the process (3) may be added and mixed at a concentration of 5 to 100 µg/ml.

In one embodiment of the present disclosure, the immortalized stem cell-derived exosome-rich conditioned medium in the process (3) may be mixed by adding 1 to 3% by weight based on a total weight% of the composition.

**In** addition, the present disclosure provides a composition for use in method for preventing or treating atopic dermatitis, in which the composition contains an immortalized stem cell-derived exosome-rich conditioned medium and a rosebud extract as active ingredients.

In one embodiment of the present disclosure, the composition may have activity in inhibiting mast cell granule secretion, inhibiting immunoglobulin IgE in serum, inhibiting histamines in serum, and improving itchiness.

In addition, the present disclosure provides a composition for use in method for preventing or treating pigmentation diseases, in which the pharmaceutical composition contains an immortalized stem cell-derived exosome-rich conditioned medium and a rosebud extract as active ingredients.

In one embodiment of the present disclosure, the pigmentation disease may be one or more of freckles, senile spots, chloasma, small brown spots, brown or black moles, sunshine pigment spots, cyanic melisma, hyperpigmentation after drug use, adverse sequelae following tissue sclerotherapy, gravidic chloasma, melasma in women taking oral contraceptives, hyperpigmentation after inflammation caused by lesions or skin inflammation like excoriation and burn, phototoxic reactions, or other similar small, fixed pigmented lesions.

### [Advantageous Effects]

The composition containing the immortalized stem cell-derived exosome-rich conditioned medium and the rosebud extract as active ingredients, according to the present disclosure, has excellent activity in inhibiting mast cell granule secretion, inhibiting immunoglobulin (IgE) in serum, inhibiting histamines in serum, and improving itchiness, is excellent in tyrosinase inhibitory activity and melanin production inhibitory activity, and may maximize the activity of active ingredients by overcoming the skin penetration issue of stem cell-derived ingredients, and thus may be usefully used in the manufacture of a cosmetic composition having activity in atopic dermatitis improvement, skin wrinkle improvement, and whitening as well as may also be usefully used in the manufacture of a pharmaceutical composition for the prevention or treatment of atopic dermatitis and pigmentation diseases.

### [Description of Drawings]

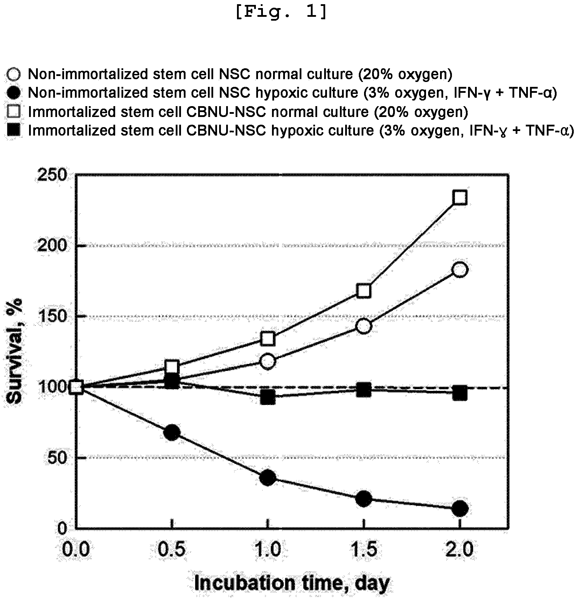

FIG. 1 shows a result of analyzing the viability according to the culture time of the non-immortalized neural stem cells (NSCs) and immortalized neural stem cell line (CBNU-NSC) under a normal oxygen concentration (20%) condition and an exosome-rich conditioned medium preparation condition (10 U/mL IFN-y, 50 ng/mL TNF-α and 3% hypoxic concentration).

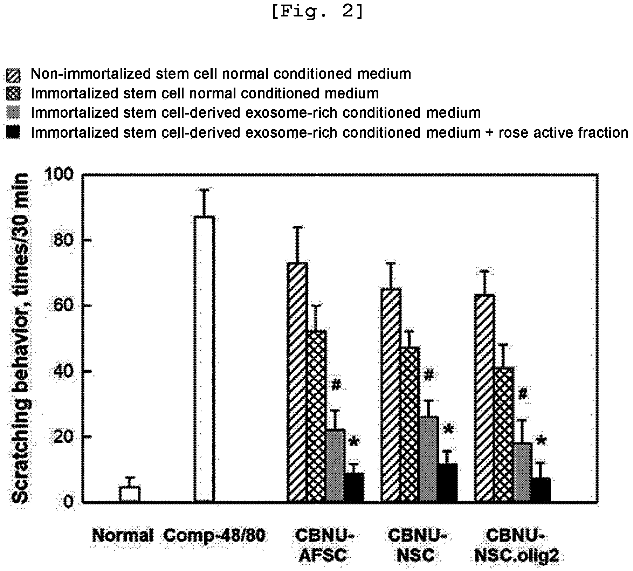

FIG. 2 shows a result of analyzing the degree of improvement after each treatment of: a non-immortalized stem cell normal conditioned medium; an immortalized stem cell normal conditioned medium; an immortalized stem cell-derived exosome-rich conditioned medium; and a mixture in which a rosebud extract is added to an immortalized stem cell-derived exosome-rich conditioned medium in itchy mice induced with Compound-48/80 (Comp-48/80), which is a substance that induces atopic dermatitis. In the graph results including FIG. 2 of the present disclosure, the hatched graph shows the group treated with the non-immortalized stem cell normal conditioned medium alone, the checkered graph shows the group treated with the immortalized stem cell normal conditioned medium alone, the gray graph shows the group treated with the immortalized stem cell-derived exosome-rich conditioned medium of the present disclosure alone, and the black graph shows the group treated with the immortalized stem cell-derived exosome-rich conditioned medium and the ethanol fraction (active fraction) of the methanol extract of rosebuds of the present disclosure together. In addition, in the graphs shown in the drawings of the present disclosure, CBUN-AFSC represents an immortalized amniotic stem cell line, CBNU-NSC represents an immortalized neural stem cell line, and CBNU-NSC.olig2 represents an immortalized oligodendrocyte progenitor cell line.

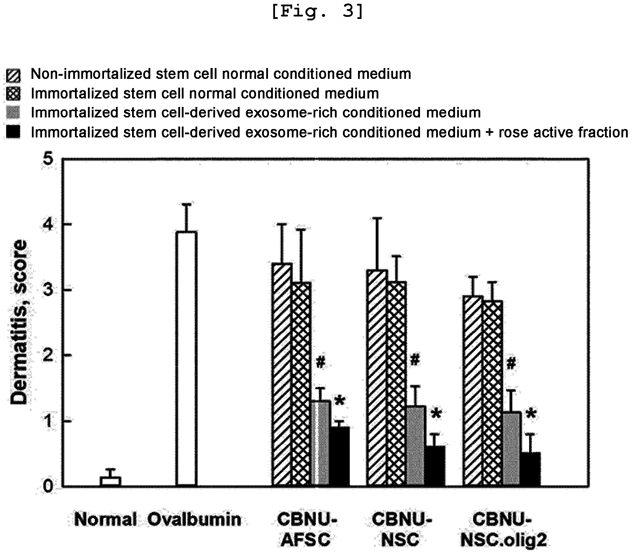

FIG. 3 shows a result of analyzing the degree of dermatitis alleviation for each group treated with a non-immortalized stem cell normal conditioned medium, an immortalized stem cell normal conditioned medium, an immortalized stem cell-derived exosome-rich conditioned medium, and a mixture of an immortalized stem cell-derived exosome-rich conditioned medium and a rosebud active fraction in mice models induced with chronic atopic dermatitis with ovalbumin.

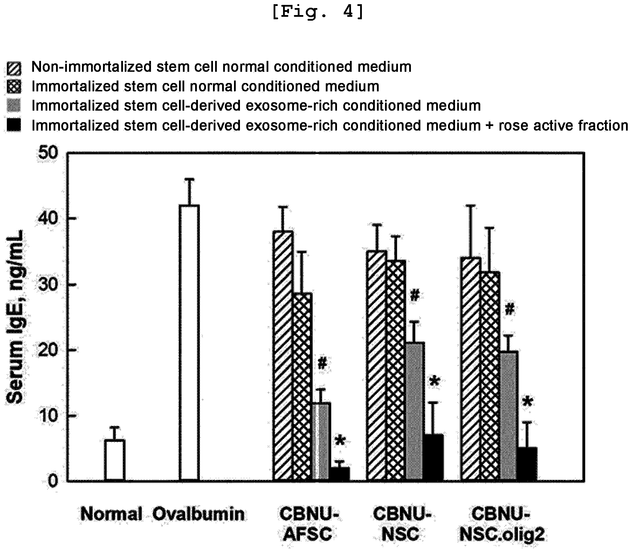

FIG. 4 shows a result of analyzing the serum immunoglobin E (IgE) inhibitory effect for each group treated with a non-immortalized stem cell normal conditioned medium, an immortalized stem cell normal conditioned medium, an immortalized stem cell-derived exosome-rich conditioned medium, and a mixture of an immortalized stem cell-derived exosome-rich conditioned medium and a rosebud active fraction in mice models induced with chronic atopic dermatitis with ovalbumin.

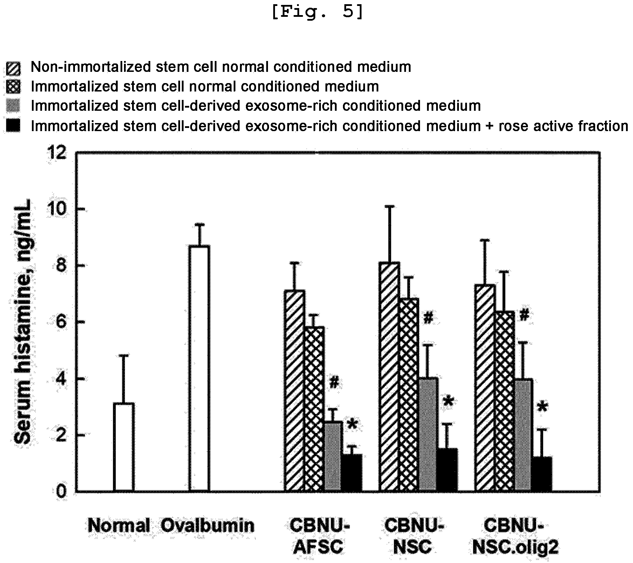

FIG. 5 shows a result of analyzing the serum histamine inhibitory effect for each group treated with a non-immortalized stem cell normal conditioned medium, an immortalized stem cell normal conditioned medium, an immortalized stem cell-derived exosome-rich conditioned medium, and a mixture of an immortalized stem cell-derived exosome-rich conditioned medium and a rosebud active fraction in models induced with chronic atopic dermatitis with ovalbumin.

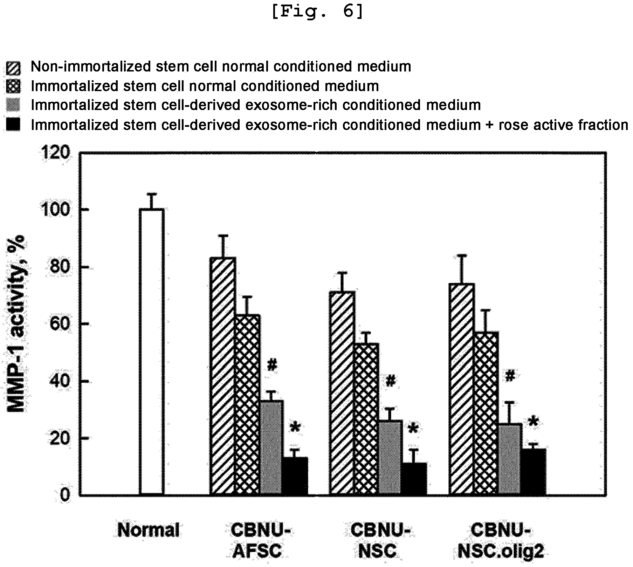

FIG. 6 shows a result of analyzing the MMP-1 activity inhibitory effect for each group treated with a non-immortalized stem cell normal conditioned medium, an immortalized stem cell normal conditioned medium, an immortalized stem cell-derived exosome-rich conditioned medium, and a mixture of an immortalized stem cell-derived exosome-rich conditioned medium and a rosebud active fraction for collagen degrading enzyme MMP-1 activity.

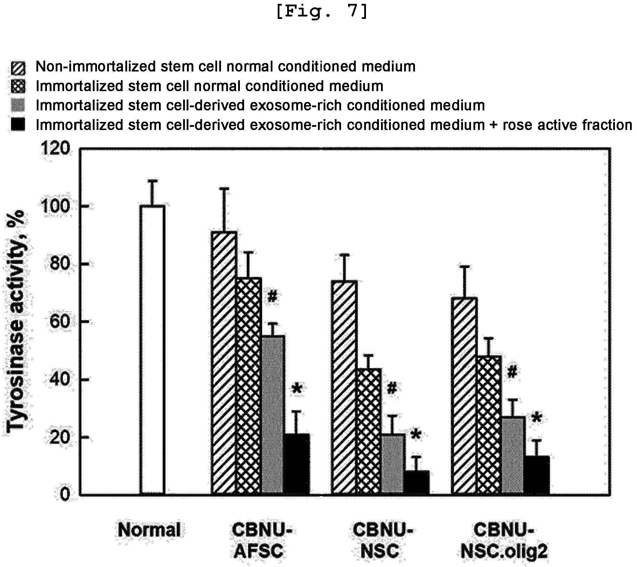

FIG. 7 shows a result of analyzing the tyrosinase activity inhibitory effect for each group treated with a non-immortalized stem cell normal conditioned medium, an immortalized stem cell normal conditioned medium, an immortalized stem cell-derived exosome-rich conditioned medium, and a mixture of an immortalized stem cell-derived exosome-rich conditioned medium and a rosebud active fraction for melanin synthetase tyrosinase activity.

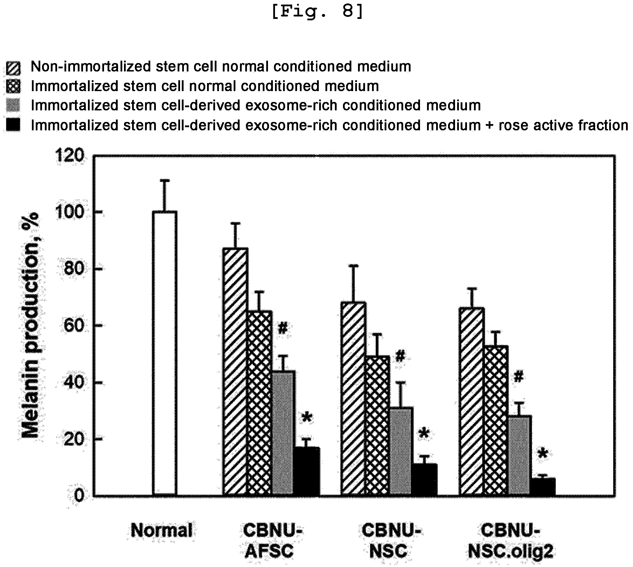

FIG. 8 shows a result of analyzing the melanin production inhibitory effect for each group treated with a non-immortalized stem cell normal conditioned medium, an immortalized stem cell normal conditioned medium, an immortalized stem cell-derived exosome-rich conditioned medium, and a mixture of an immortalized stem cell-derived exosome-rich conditioned medium and a rosebud active fraction for melanocytes.

### [Best Mode for Carrying out Invention]

In the midst of research to develop a raw material for a new functional cosmetic with excellent atopic dermatitis improvement, wrinkle improvement or skin whitening activity using a stem cell conditioned medium and a rosebud extract, the present inventors confirmed that the composition containing an immortalized stem cell-derived exosome-rich conditioned medium together with a rosebud extract as active ingredients exhibited excellent atopic dermatitis improvement effect, wrinkle improvement effect, and skin whitening effect. The present invention is as defined in the attached set of claims.

Accordingly, the present disclosure is characterized by providing a composition containing an immortalized stem cell-derived exosome-rich conditioned medium together with a rosebud extract as active ingredients.

The "stem cells" are cells that have not been differentiated into specific cells, and if necessary, cells having the ability to differentiate into all types of cells constituting the body, such as nerves, skin, blood, muscle, bone, and cartilage. Recently, research to use stem cells in various industrial fields such as functional cosmetics and pharmaceuticals is being attempted.

Research on the use of stem cells in the manufacture of functional cosmetics is also being actively carried out. Most of the research being conducted so far is the result of obtaining wrinkle improvement and whitening effects by subcutaneously injecting stem cells themselves. There is no confirmed result using a stem cell conditioned medium. **In** particular, there is an issue that the active ingredient in the stem cell conditioned medium is small and mainly composed of protein, so that it does not penetrate the skin well.

Accordingly, the present inventors conducted a study to identify the optimal conditions for activating stem cells and secreting a large amount of exosomes without destroying the protein-containing exosomes secreted from the stem cells, as a method for improving the skin penetration rate of these components in order to effectively use the conditioned medium containing the stem cell-derived active ingredients.

In addition, in the present disclosure, a condition capable of secreting a large amount of exosomes using the stem cells was established and the stem cells were cultured. In the present disclosure, it was confirmed that the viability of stem cells was significantly reduced in the condition of exosome secretion in large amounts, and thus it was confirmed that there is a limit to using the general stem cells themselves.

Therefore, as a way to overcome this, the present inventors manufactured "immortalized stem cells" from stem cells and devised a method for using them. Specifically, the present inventors established an immortalized cell line by introducing v-myc oncogene into each stem cell so that the proliferative ability does not decrease and a high viability can be maintained even during continuous subculture while maintaining the characteristics of the stem cells, and identified that the immortalized stem cell line of the present disclosure could secrete a large amount of exosomes, showing a high viability without dying even under the condition of exosome secretion in large amounts of the present disclosure, unlike general stem cells.

Accordingly, the present disclosure is characterized in that the composition having excellent activity in atopic dermatitis improvement, wrinkle improvement or skin whitening contains an immortalized stem cell-derived exosome-rich conditioned medium of the present disclosure as an active ingredient.

On the other hand, "exosomes" are small-sized (30-150 nm) vesicles containing sophisticated RNA and protein transport materials, and are membrane-structured vesicles secreted from various types of cells. The exact molecular mechanisms and functions of exosome secretion, absorption, and composition have not yet been sufficiently studied. However, exosomes have been known to play a role in delivering membrane components, proteins, and RNAs that bind to other cells and tissues.

Accordingly, the present inventors have identified the conditions that a large amount of exosomes can be secreted from the stem cells in a more effective way compared to the conventional art of capturing the active ingredient with artificial liposomes composed of lipids, in order to increase the skin absorption rate of the active ingredient in a stem cell conditioned medium. When these conditions are applied, it was found that issues such as a separate treatment process according to the liposome grafting method, low active ingredient collection rate, contamination of external substances, and deterioration of the conditioned medium components when collecting liposomes could be solved.

Accordingly, the immortalized stem cells according to the present disclosure may secrete a large amount of exosomes without destroying the stem cell-derived exosomes, and may be cultured under the culture conditions established in the present disclosure in which apoptosis of immortalized stem cells is not induced. The culture conditions include treating IFN-y and TNF-α together in immortalized stem cells or a culture medium containing immortalized stem cells, and culturing the same under hypoxic conditions. Preferably, IFN-y may be treated at a concentration of 1 to 10 U/mL, TNF-α may be treated at a concentration of 3 to 100 ng/mL, and cultured for 12 to 72 hours under hypoxic conditions of 1 to 5%.

In particular, while studying a promoter capable of activating immortalized stem cells to promote the secretion of exosomes, the present inventors confirmed that among many candidate substances, when IFN-y and TNF-α were simultaneously treated, exosome secretion was most effectively promoted. It was found to be preferable that the conditions for maximally secreting exosomes were when IFN-y was treated in an amount of 1 to 10 U/mL, and TNF-α was treated in an amount of 3 to 100 ng/mL.

When the amount of IFN-y and TNF-α treatment exceeds the above-described range, it may cause damage to immortalized stem cells and cause the cells to fail to function normally, while when treated at a concentration that does not fall within the above range, the amount of exosome secretion is insufficient.

Stem cells, like macrophages, are activated and migrated by TNF-α after being sensitized by IFN-γ. Activated macrophages secrete active oxygen radicals to attack microorganisms and tumors invaded into the body, whereas activated stem cells secrete growth factors (GF) and neurotrophic factors (NF) to protect and regenerate tissues. These GF/NF proteins are known to be secreted by being captured in exosomes. Accordingly, in the present disclosure, it was confirmed that the condition that a large amount of GF/NF-containing exosomes could be obtained from immortalized stem cells was when IFN-y is treated with 1 to 10 U/mL and TNF-α is treated with 3 to 100 ng/mL at the same time.

In addition, it was confirmed that culture under oxygen concentration significantly lower than normal concentration (about 20%) was required as another requirement for inducing a large amount of exosome secretion from immortalized stem cells of the present disclosure, and it was found that it was preferable to culture for 12-72 hours under hypoxic conditions of 1 to 5% after IFN-y and TNF-α treatment. In this connection, when the oxygen concentration condition is less than 1%, it may cause issues in normal proliferation and growth of cells, and when it exceeds 5%, exosome secretion may not be sufficient. Accordingly, it is preferable to carry out under hypoxic conditions of 1-5%, and more preferably, it is better to culture at an oxygen concentration of 3%.

In addition, in the method according to the present disclosure, the culture medium of the immortalized stem cells may be any medium for culturing stem cells, and in one embodiment of the present disclosure, DMEM (*Dulbecco's modified Eagle's medium*) was used.

In addition, the immortalized stem cells treated with IFN-y (1 to 10 U/mL) and TNF-α (3 to 100 ng/mL) in the present disclosure may be preferably used in the number of 1 × 10⁴ to 1 × 10⁷ cells.

As described above, when immortalized stem cells are cultured under the conditions of the present disclosure, the secretion of exosomes containing physiologically active substances such as cell proliferation, differentiation, and regeneration-related genes, proteins, and growth factors secreted during the proliferation of immortalized stem cells may be promoted and enhanced, and may significantly increase the secretion and production of exosomes compared to the general method for obtaining a stem cell culture medium (in other words, the method not performing culture under IFN-y, TNF-α and hypoxic concentration conditions).

In addition, in the case of non-immortalized stem cells, since the cell viability is very low under the treatment of IFN-y and TNF-α and under the conditions of hypoxic concentration, a sufficient amount of exosomes cannot be obtained, so only when using the immortalized stem cells of the present disclosure, a large amount of exosomes, or a large amount of exosome-containing conditioned medium (in other words, an exosome-rich conditioned medium) may be obtained.

The immortalized stem cells that may be used in the present disclosure may be any immortalized stem cells obtained by immortalizing stem cells, but may be immortalized amniotic stem cells, immortalized amnion stem cells, immortalized placental stem cells, immortalized umbilical cord blood stem cells, immortalized adipose stem cells, immortalized bone marrow stem cells, immortalized neural stem cells, and immortalized oligodendrocyte progenitor cells.

Preferably, the immortalized stem cells may be immortalized amniotic stem cells, immortalized neural stem cells or immortalized oligodendrocyte progenitor cells, and more preferably, the immortalized amniotic stem cells may be immortalized amniotic stem cells CBNU-AFSC cell line (Accession No.: KCTC12634BP), the immortalized neural stem cells may be immortalized neural stem cells, CBNU-NSC cell line (Accession No.: KCTC12635BP), and the immortalized oligodendrocyte progenitor cells may be immortalized oligodendrocyte progenitor cells, CBNU-NSC.olig2 cell line (Accession No.: KCTC12636BP).

Moreover, the composition having excellent activity in atopic dermatitis improvement, wrinkle improvement or skin whitening of the present disclosure contains a rosebud extract as another active ingredient.

The "rosebud extract" of the present disclosure may be obtained by extraction and separation from a rosebud using a method for extraction and separation known in the pertinent field, and the extract defined in the present disclosure may be extracted from rosebuds by using an appropriate solvent. The rosebud extract includes, for example, all of the crude extract of rosebuds, the polar solvent-soluble extract, or the non-polar solvent-soluble extract.

As a suitable solvent for obtaining the extract from the rosebuds, any industrially usable organic solvent may be used, and water or an organic solvent may be used. For example, various solvents such as purified water, alcohols having 1 to 4 carbon atoms such as methanol, ethanol, propanol, isopropanol and butanol, acetone, ether, benzene, chloroform, ethyl acetate, methylene chloride, hexane, and cyclohexane may be used alone or in combination. Preferably, purified water (water) or methanol may be used, and in an embodiment of the present disclosure, methanol was used.

The extraction method may use any one selected from methods including hot water extraction, chilling extraction, reflux cooling extraction, solvent extraction, steam distillation, ultrasonic extraction, elution, pressing, and the like. Further, the desired extract may additionally perform a general fractionation process and be purified using a general purification method.

Preferably, the extract of the present disclosure may be a solvent fraction of rosebuds obtained by adding and extracting ethanol, butanol, or ethyl acetate as a solvent to the methanol extract of rosebuds.

More preferably, the rosebud extract of the present disclosure may be obtained by adding methanol to a dried and pulverized rosebud, sonicating for 1 to 2 hours at a temperature of 28 to 30°C, drying the obtained extract at a temperature of 40 to 50°C, and then adding ethanol, butanol, or ethyl acetate thereto, and performing a fractionation at 50 to 55°C for 1 to 2 hours.

In particular, in the present disclosure, in order to effectively extract a large amount of active ingredients having activity in atopic dermatitis improvement, wrinkle improvement or whitening from rosebuds, ultrasonic treatment was performed on the methanol extract of rosebuds. When the extraction is performed at a temperature of less than 28°C during ultrasonic treatment for less than 1 hour, the extraction of the active ingredient from the rosebuds is insufficient. On the other hand, when the extraction is performed at a temperature exceeding 30°C for more than 2 hours, the active ingredient loses its activity or becomes denatured and thus cannot exert its intended function.

In one embodiment of the present disclosure, 70% ethanol, 70% butanol or 70% ethyl acetate was used to obtain a solvent fraction from the methanol extract of the rosebuds.

In addition, as the rosebuds, a rosebud of a rose selected from the group consisting of Icewing (*Rosa hybrida* Icewing), Onnuri (*Rosa hybrida* Onnuri), Colorado (*Rosa hybrida* Colorado) and rose vines (*Rosa multiflora* Platyphylla thory) may be used.

The immortalized stem cell-derived exosome-rich conditioned medium, which is an active ingredient contained in the composition of the present disclosure, may be contained in an amount of 1 to 3% by weight based on a total weight% of the composition, and the rosebud extract may be contained at a concentration of 5 to 100 µg/ml.

When the immortalized stem cell-derived exosome-rich conditioned medium and the rosebud extract are contained in less than the above range with respect to the composition of the present disclosure, atopic dermatitis improvement, skin wrinkle improvement or whitening activity is insufficient, whereas when the immortalized stem cell-derived exosome-rich conditioned medium and the rosebud extract are contained in excess, not only is it uneconomical because the enhancement of the effect proportional to the amount added does not appear, but also the activities are rather lowered by the interaction between the two active ingredients. Hence, it is important that each active ingredient is contained in the composition in the range described above.

In one embodiment of the present disclosure, in order to analyze whether activity in atopic dermatitis improvement, wrinkle improvement or whitening according to the treatment of the immortalized stem cell-derived exosome-rich conditioned medium and the rosebud extract, after treatment with atopic dermatitis-induced mice with a mixed solution containing a rosebud extract (active fraction) and an immortalized stem cell-derived exosome-rich conditioned medium of the present disclosure, the degree of improvement was confirmed. It was found that the treatment with the immortalized stem cell-derived exosome-rich conditioned medium together with the rosebud extract effectively alleviated histamine secretion and skin inflammation compared to the case of treatment with each thereof alone, thus having the significantly superior improvement effect of atopic dermatitis. It was found that the group using the immortalized stem cell conditioned medium of the present disclosure had a better improvement effect than the group using the non-immortalized stem cell conditioned medium.

Moreover, it was confirmed that the case where the immortalized stem cell-derived exosome-rich conditioned medium was treated together with the rosebud extract had a significantly superior effect compared to the case where each of them was treated alone also in terms of inhibition of MMP-1 (matrix metalloproteinase-1), a collagen degrading enzyme that mainly causes skin wrinkles, and tyrosinase activity, which causes pigmentation, and inhibition of melanin production.

Accordingly, the composition containing the immortalized stem cell-derived exosome-rich conditioned medium and rosebud extract as active ingredients of the present disclosure has activity in atopic dermatitis improvement, skin wrinkle improvement or whitening.

A composition according to the present disclosure may be one or more formulations selected from the group consisting of skin lotion, skin softener, skin toner, astringent, lotion, milk lotion, moisture lotion, nutrition lotion, massage cream, nutrition cream, moisture cream, hand cream, foundation, essence, nutrition essence, pack, soap, cleansing foam, cleansing lotion, cleansing cream, body lotion, body cleanser, face wash, treatment, beauty liquids, beauty packs, ointments, gels, liniments, liquids, patches, and sprays. However, may be prepared in the formulation of a conventional cosmetic composition.

General additives in the cosmetic field may be added. Examples of general additives in the cosmetic field may be one or more selected from the group consisting of antibiotics, binders, disintegrants, diluents, lubricants, stabilizers, preservatives, fragrances, oil, water, surfactants, humectants, lower alcohols, thickeners, chelating agents, pigments and preservatives.

In addition, the present disclosure may provide a method for preparing a composition having activity in atopic dermatitis improvement, skin wrinkle improvement and whitening.

The method preferably includes: (1) simultaneously treating TNF-α and interferon gamma (IFN-y) in a culture medium containing immortalized stem cells, and culturing the same under a low oxygen concentration condition of 1 to 5% for 12 to 72 hours to obtain an immortalized stem cell-derived exosome-rich conditioned medium; (2) adding any one of solvents selected from the group consisting of ethanol, butanol and ethyl acetate to a methanol ultrasonic extract obtained by adding methanol to dried and pulverized rosebuds and sonicating the same, followed by extraction and fractionation to obtain a solvent fraction for a methanol extract of rosebuds; and (3) mixing the immortalized stem cell-derived exosome-rich conditioned medium obtained in the process (1) and the solvent fraction for a methanol extract of rosebuds obtained in the process (2).

The immortalized stem cells that may be used in the above method have the same as the contents of the immortalized stem cells described above, and most preferably, the immortalized stem cells may use immortalized amniotic stem cells, CBNU-AFSC cell line (Accession No.: KCTC12634BP), immortalized neural stem cells, CBNU-NSC cell line (Accession No.: KCTC12635BP), or immortalized oligodendrocyte progenitor cells, CBNU-NSC.olig2 cell line (Accession No.: KCTC12636BP).

In addition, in the process (1), the TNF-α may be treated at a concentration of 3 to 100 ng/ml, and the interferon gamma (IFN-y) may be treated at a concentration of 1 to 10 U/ml. The solvent fraction for the methanol extract of rosebuds obtained in the process (2) in the immortalized stem cell-derived exosome-rich conditioned medium in the process (3) may be a fraction obtained by using ethanol. In addition, the solvent fraction for the methanol extract of rosebuds in the process (3) may be added and mixed at a concentration of 5 to 100 µg/ml. The immortalized stem cell-derived exosome-rich conditioned medium may be mixed by adding 1 to 3% by weight based on a total weight% of the composition.

Moreover, the present disclosure may provide a composition for use in a method for preventing or treating atopic dermatitis, in which the pharmaceutical composition contains an immortalized stem cell-derived exosome-rich conditioned medium and a rosebud extract as active ingredients.

The composition of the present disclosure containing an immortalized stem cell-derived exosome-rich conditioned medium and a rosebud extract as active ingredients has excellent characteristics of inhibiting mast cell granule secretion, inhibiting immunoglobulin IgG in serum, inhibiting histamine in serum, and improving itchiness, and thus may effectively prevent, improve and treat atopic dermatitis.

In addition, the present disclosure may provide a composition for use in method for preventing or treating pigmentation diseases, in which the pharmaceutical composition contains an immortalized stem cell-derived exosome-rich conditioned medium and a rosebud extract as active ingredients.

In one embodiment of the present disclosure, when the composition of the present disclosure containing an immortalized stem cell-derived exosome-rich conditioned medium and a rosebud extract as active ingredients may effectively inhibit and prevent the inhibition of melanocytes in the skin when applied to the hyperpigmented skin. More specifically, it is possible to effectively cure diseases and lesions related to pigmentation by removing melanin pigmented on the skin through inhibition of melanin synthesis and tyrosinase activity.

The pigmentation diseases may include, all diseases and lesions that may be caused by abnormally generated melanin in the skin, and preferably, the pigmentation diseases may be one or more selected from the group consisting of freckles, senile spots, chloasma, small brown spots, brown or black moles, sunshine pigment spots, cyanic melisma, hyperpigmentation after drug use, adverse sequelae following tissue sclerotherapy, gravidic chloasma, melasma in women taking oral contraceptives, hyperpigmentation after inflammation caused by lesions or skin inflammation like excoriation and burn, phototoxic reactions, or other similar small, fixed pigmented lesions.

The composition may be formulated by adding a pharmaceutically acceptable carrier, and for information on formulation, please refer to Remington's Pharmaceutical Science (latest edition), Mack Publishing Company, Easton PA.

The pharmaceutically acceptable carrier refers to one that is commonly used in the preparation of a pharmaceutical composition by those skilled in the art of a pharmaceutical invention. For example, pharmaceutically acceptable carriers include lactose, dextrose, sucrose, sorbitol, mannitol, xylitol, erythritol, maltitol, starch, acacia rubber, alginate, gelatin, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate and mineral oil. In addition, pharmaceutically acceptable carriers include diluents or excipients such as fillers, extenders, binders, wetting agents, disintegrants, surfactants, and the like.

For example, the pharmaceutical carriers and/or excipients suitable for topical administration are preferably, according to the present disclosure, conventional carriers and/or excipients known to those skilled in the pertinent technical field in connection with pharmaceutical preparations for dermal administration (=dermatologicals).

A solid preparation for oral administration includes tablets, pills, powders, granules, capsules, and the like, and such a solid preparation may be compounded by mixing the pharmaceutical composition of the present disclosure with at least one or more excipients, for example, starch, calcium carbonate, sucrose, lactose or gelatin, etc. Furthermore, in addition to simple excipients, lubricants such as magnesium, stearate and talc may also be used.

A liquid preparation for oral administration includes suspensions, internal solutions, emulsions, syrups, and the like, where various excipients, for example, wetting agents, sweeteners, aromatics and preservatives may be included other than water or liquid paraffin as simple diluents that are commonly used.

Examples which may be mentioned for dermal administration include dusting powders, emulsions, suspensions, oils, sprays, ointments, greasy ointments, cream pastes, gels, foams, or carriers and/or excipients suitable for preparing solutions and transdermal therapeutic system (TTS).

A topical pharmaceutical preparation of the present disclosure may be in a semi-solid dosage form, and in particular, there are ointments (solution ointments, suspension ointments), creams, gels or pastes. Mainly used in the oil phase are fatty alcohols such as lauryl alcohol, cetyl alcohol, stearyl alcohol, fatty acids such as palmitic or stearic acid, liquid or solid paraffin or ozokerite, liquid to solid waxes, such as isopropyl myristate, natural fats or some synthetic fats such as coconut fatty acid triglycerides, hydrogenated oils such as hydrogenated peanut or castor oil, or fatty acid partial esters of glycerol such as glycerol monostearate or glycerol distearate. Suitable emulsifiers include surfactants, for example nonionic surfactants, for example fatty acid esters of polyalcohols or ethylene oxide adducts thereof, such as polyglycerol fatty acid esters or polyoxyethylene sorbitan fatty acid esters, sorbitan fatty acid esters, such as sorbitan oleate and/or sorbitan isostearate and the like, isostearates, sterols, or polyoxyethylene fatty alcohol ethers or fatty acid esters such as anionic surfactants such as alkali metal salts of fatty alcohol sulfonates, such as sodium lauryl sulfate, sodium cetyl sulfate or sodium stearyl sulfate, which are usually used in the presence of the fatty alcohol, for example cetyl alcohol or stearyl alcohol. In particular, it is possible to add an agent for preventing drying of the cream, for example, a polyalcohol, such as glycerol, sorbitol, propylene glycol and/or polyethylene glycol, to an aqueous phase, or add a preservative, flavoring, etc. to an aqueous phase.

A pharmaceutical preparation of the present disclosure may be an anhydrous ointment, suitable for topical use, and contain paraffin, particularly low-viscosity paraffin, which is liquid at body temperature, or the natural or partially synthetic fat, for example, coconut fatty acid triglyceride, hydrogenated oils such as hydrogenated peanut or castor oil, fatty acid partial esters of glycerol such as glycerol monostearate and distearate, silicones such as polymethylsiloxanes such as hexamethyldisiloxane or octamethyltrisiloxane. For example, it may contain fatty alcohols that are associated with aqueous creams and increase water absorption capacity, as well as sterols, wool waxes, other emulsifiers and/or other additives.

The applied amount of the active ingredients contained in a pharmaceutical composition varies depending on the condition and weight of a patient, the severity of disease, drug form, administration route and period, but may be appropriately selected in some cases. For example, the active ingredient may be administered at a dose of 0.0001 to 1,000 mg/kg per day, preferably 0.1 to 1,000 mg/kg, and the application may be applied once or dividedly several times a day. In addition, the pharmaceutical composition may include 0.0001 to 50% by weight of the active ingredients of the present disclosure with respect to a total weight of the composition.

A pharmaceutical composition may be applied to mammals such as humans by various routes, for example, by transdermal, oral, intravenous, intramuscular, or subcutaneous injection.

In addition, a pharmaceutical composition of the present disclosure may contain one or more active ingredients that improve, alleviate, treat or prevent skin pigmentation, in addition to the active ingredients.

In addition, a pharmaceutical composition of the present disclosure may be used alone or in combination with methods using surgery, hormone therapy, drug treatment and biological response modifiers for the improvement, alleviation, treatment or prevention of skin pigmentation.

Moreover, the present disclosure may provide a food composition having activity in atopic dermatitis improvement, skin wrinkle improvement, and whitening, in which the food composition contains an immortalized stem cell-derived exosome-rich conditioned medium and a rosebud extract as active ingredients.

The food composition of the present disclosure may contain various flavoring agents or natural carbohydrates as additional ingredients, as in a conventional food composition, in addition to containing an immortalized stem cell-derived exosome-rich conditioned medium and a rosebud extract as active ingredients.

Examples of the above-mentioned natural carbohydrates include monosaccharides such as glucose and fructose; disaccharides such as maltose and sucrose; and polysaccharides such as conventional sugars such as dextrin and cyclodextrin, and sugar alcohols such as xylitol, sorbitol, and erythritol. The above-mentioned flavoring agents may advantageously use natural flavoring agents (thaumatin), stevia extract (for example, rebaudioside A, glycyrrhizin, etc.) and synthetic flavoring agents (saccharin, aspartame, etc.).

The food composition of the present disclosure may be formulated in the same manner as the pharmaceutical composition and used as a functional food or added to various foods. Foods to which the composition of the present disclosure may be added include, for example, beverages, meat, chocolate, foods, confectionery, pizza, ramen, other noodles, gums, candy, ice cream, alcoholic beverages, vitamin complexes and health supplements.

In addition, in addition to the active ingredients of the present disclosure, the food composition may contain various nutrients, vitamins, minerals (electrolytes), flavorings such as synthetic flavorings and natural flavorings, colorants and enhancers (cheese, chocolate, and the like), pectic acid and salts thereof, alginic acid and salts thereof, organic acids, protective colloid thickeners, pH adjusting agents, stabilizers, preservatives, glycerin, alcohols, and carbonating agents used in carbonated beverages. In addition, the food composition of the present disclosure may contain natural fruit juice and pulp for the production of fruit juice drinks and vegetable drinks.

In addition, the present disclosure may provide a health functional food having activity in atopic dermatitis improvement, skin wrinkle improvement, and whitening, in which the health functional food contains an immortalized stem cell-derived exosome-rich conditioned medium and a rosebud extract as active ingredients.

The health functional food of the present disclosure may be manufactured and processed in the form of tablets, capsules, powders, granules, liquids, pills, and the like.

In the present disclosure, the term "health functional food" refers to food manufactured and processed using ingredients or components having functional properties useful for the human body according to Act No. 6727 of the Health Functional Food, and means ingestion for the purpose of obtaining useful effects for health purposes such as controlling nutrients or physiological effects on the structure and function of the human body.

The health functional food of the present disclosure may include conventional food additives and its suitability as a food additive is determined by the specifications and standards for the relevant item in accordance with General Regulations and General Test Methods of Korean Food Additives Codex approved by the Ministry of Food and Drug Safety, unless otherwise specified.

Examples of the items published in the above-mentioned "Korean Food Additives Codex" include chemical synthetics such as ketones, glycine, potassium citrate, nicotinic acid, and cinnamic acid, natural additives such as persimmon extract, licorice extract, crystalline cellulose, kaoliang color and guar gum, mixed preparations such as L-sodiumglutamate preparation, alkaline agents for noodles, preservative formulation and a tar color formulation and the like.

For example, the health functional food in the form of tablets may be granulated by a conventional method with a mixture of the active ingredients of the present disclosure with an excipient, binder, disintegrant and other additives, followed by compression molding by putting a lubricant, etc. or direct compression molding of the mixture. In addition, the health functional food in the form of tablets may contain bitters, or the like, if necessary.

Among health functional foods in the form of capsules, hard capsules may be prepared by filling a mixture of the active ingredients of the present disclosure with additives such as excipients in a conventional hard capsule, and soft capsules may be prepared by filling a mixture of the active ingredients of the present disclosure with additives such as an excipient in a capsule base such as gelatin. The soft capsules may contain a plasticizer such as glycerin or sorbitol, a colorant, a preservative, and the like, if necessary.

The health functional food in the form of pills may be prepared by molding a mixture of the active ingredients of the present disclosure with an excipient, a binder, a disintegrant, etc. by a conventionally known method, and if necessary, it may be coated with white sugar or other coating agents. Alternatively, the surface may be coated with a material such as starch or talc.

The health functional food in the form of granules may be prepared in granular form by a conventionally known method with a mixture of the active ingredients of the present disclosure with an excipient, binder, disintegrant, and the like. If necessary, it may contain fragrance ingredients, bitters, and the like.

The health functional food may be beverages, meat, chocolate, foods, confectionery, pizza, ramen, other noodles, gums, candy, ice cream, alcoholic beverages, vitamin complexes and health supplements.

### [Mode for Carrying out Invention]

Hereinafter, the present disclosure will be described in more detail through examples. These examples are for illustrating the present disclosure in more detail.

### <Preparation Examples>

### ① Preparation of Immortalized Stem Cells

The following cells were used as the immortalized stem cells used in the experiments of the following examples of the present disclosure. First, immortalized amniotic stem cells, immortalized neural stem cells and immortalized oligodendrocyte progenitor cells are cell lines presently prepared and established as immortalized cell lines by the present inventors, and each immortalized cell line was given an accession number from the microbial depository. Specifically, as each immortalized stem cell used in the following examples, immortalized amniotic stem cells, CBNU-AFSC cell line (Accession No.: KCTC12634BP; Korean Patent No. 10-1719274), immortalized neural stem cells, CBNU-NSC cell line (Accession No.: KCTC12635BP; Korean Patent No. 10-1719274), and immortalized oligodendrocyte progenitor cells, CBNU-NSC.olig2 cell line (Accession No.: KCTC12636BP; Korean Patent No. 10-1740357) were used, respectively. For the method of preparing the immortalized cell lines, reference may be made to the contents of the registered patent for each cell line. In addition, as the non-immortalized stem cells used as comparative examples in the examples below, each primary cultured cell before immortalization of neural stem cells and immortalized oligodendrocyte progenitor cells was used.

### ② Culturing of Immortalized Stem Cells and Obtaining an Exosome-Rich Conditioned Medium

Each of the immortalized stem cells (1 × 10⁶ cells/mL) and the non-immortalized stem cells prepared in Preparation Example ① were inoculated into Dulbecco's modified Eagle's medium (DMEM), and cultured for 24 hours at a temperature of 37°C in a 5% carbon dioxide (CO₂) supply incubator. Then, in order to obtain an exosome-rich conditioned medium (ERCM) from each immortalized stem cell, IFN-y (10 U/mL) and TNF-α (50 ng/mL) were simultaneously treated in the culture medium, the oxygen concentration was lowered to 3%, cultured for 24 hours, and each conditioned medium was collected and filtered. Thereafter, they were concentrated 10 times at a low temperature to obtain an immortalized stem cell-derived exosome-rich conditioned medium according to the present disclosure. The normal conditioned medium (CM) used as a control group was cultured for 24 hours in a state of maintaining an oxygen (O₂) concentration of 20% at a temperature of 37°C, which is a general cell culture condition, for each of the immortalized and non-immortalized stem cells used above. Thereafter, the conditioned medium was collected and filtered through a filter, and the conditioned medium concentrated 10 times at a low temperature was used.

In addition, in the process of obtaining a conditioned medium containing exosomes from each of the non-immortalized stem cells and the immortalized stem cells as a comparison group, the exosome-containing conditioned medium obtained by the same method described above was used except that only TNF-α (50 ng/mL) was treated and the oxygen (O₂) concentration was maintained at 21%.

### (3) Preparation of Extracts from Rosebuds

In order to obtain a rosebud extract having antioxidant, atopic dermatitis, wrinkle and whitening improvement effects, the present inventors first selected about 20 species with a high content of antioxidative components for many rose varieties, and then finally selected four species of Icewing (*Rosa hybrida* Icewing), Onnuri (*Rosa hybrida* Onnuri), Colorado (*Rosa hybrida* Colorado) and rose vines (*Rosa multiflora* Platyphylla thory), which have the best antioxidant, atopic dermatitis, wrinkle and whitening improvement effects. Roses eco-friendly grown in Nuri Nongsan, Cheonan were supplied and used. In order to obtain extracts from the four species of roses, first, the rosebuds were dried, the dried rosebuds were pulverized, and 80% methanol corresponding to 10 times the weight of the pulverized sample was added respectively, and then ultrasonic extraction was performed at 28°C for 1 hour using an ultrasonic extraction device. Thereafter, the ultrasonic extract was dried at 40°C using a rotary vacuum concentrator, and the methanol-extracted dried product was extracted again with 70% ethanol, 70% butanol or 70% ethyl acetate at 50°C for 1 hour to obtain the solvent-extracted fractions of each rosebud.

### <Example 1>

### Analysis of Viability of Immortalized Stem Cells and Non-Immortalized Stem Cells of Present Disclosure under Culture Conditions for Obtaining Exosome-Rich Conditioned Medium

The present inventors measured the viability of each stem cell (immortalized stem cells and non-immortalized stem cells) in the case of culturing the stem cells of the preparation example under the culture conditions (10 U/mL IFN-y, 50 ng/mL TNF-α, and 3% hypoxic concentration) to obtain an exosome-rich conditioned medium from each stem cell.

As a result, as shown in FIG. 1, both non-immortalized neural stem cells and immortalized neural stem cell lines showed rapid cell proliferation at 183% and 234% for 2 days during normal oxygen concentration (20%) culture. In contrast, in the case of cultured under exosome-rich conditioned medium production conditions, in the case of non-immortalized neural stem cells, only 15% of the cells survived and most of the cells died. On the other hand, the immortalized neural stem cell line of the present disclosure showed viability of 96%. Hence, it was found that most cells did not die and showed excellent resistance even under hypoxic conditions and showed a high cell viability. These results showed similar results in non-immortalized neural stem cells, non-immortalized amniotic stem cells, and non-immortalized oligodendrocyte progenitor cells.

Accordingly, through these results, it was found that most of the non-immortalized stem cells were induced to apoptosis under the culture conditions of 10 U/mL IFN-y, 50 ng/mL TNF-α and 3% hypoxic concentration to obtain an exosome-rich conditioned medium, and that the conditioned medium of non-immortalized stem cells could not be obtained, and only when the immortalized stem cells of the present disclosure were cultured under the above conditions, an exosome-rich conditioned medium could be obtained. Accordingly, it was confirmed that the use of immortalized stem cells is an important factor in order to obtain an exosome-rich conditioned medium under the above conditions.

### <Example 2>

### Antioxidant Activity Analysis of Extract of Rosebuds of Present Disclosure

In order to analyze the antioxidant activity of each solvent-extracted fraction of the rosebuds obtained in Preparation Example ③ above, 1,1-diphenyl-2-picrylhydrazyl (DPPH) radical scavenging activity analysis was performed. To this end, 0.8 mL of 0.2 mM DPPH solution was added to 0.2 mL of each solvent-extracted fraction of rosebuds at a concentration of 50 µg/mL, and left at room temperature for 30 minutes, and then absorbance was measured at 520 nm. The electron donating ability of DPPH radical scavenging activity was expressed as mg of ascorbic acid in 1 g of the sample as the difference in absorbance between the group to which the rosebud fraction was added and the group to which the rosebud fraction was not added.

As a result, the DPPH scavenging antioxidant activities against the ethanol fractions of the methanol extracts of Icewing, Onnuri, Colorado and rose vines were confirmed as 0.69 ± 0.03, 0.72 ± 0.03, 0.52 ± 0.02 and 0.58 ± 0.03 mg ascorbic acid equivalent/g, respectively. These results showed that the antioxidant activities of Icewing, Onnuri, Colorado and rose vines were higher than 0.25 to 0.44 mg/ascorbic acid equivalent/g of other varieties. In addition, as a result of antioxidant analysis of each solvent-extracted fraction for the methanol extracts of Icewing, Onnuri, Colorado and rose vines, it was confirmed that the ethanol fraction had the best antioxidant activity compared to the butanol fraction and the ethyl acetate fraction from the methanol extract.

Then, the present inventors confirmed through the examples whether activity in atopic dermatitis improvement, whitening and wrinkle improvement was shown when the solvent-extracted fraction of the rosebuds of the present disclosure, which was identified to have excellent antioxidant activity, was used together with the immortalized stem cell-derived exosome-rich conditioned medium of the present disclosure.

### <Example 3>

### Analysis of Inhibitory Efficacy of Mast Cell Granule Secretion according to Treatment of Immortalized Stem Cell-Derived Exosome-Rich Conditioned Medium and Rosebud Extract of Present Disclosure

The secretion of mast cell granules, which is the cause of atopic dermatitis, was analyzed by measuring β-hexosaminidase, an index of granules. Specifically, the mast cell line RBL-2H3 cells were seeded at 2.5 × 10⁵ cells/well, treated with IgE at 1 ng/mL for 12 hours, and then washed. Then, in cultured cells, each 1% of the 10-fold concentrate (final concentration in the medium, v/v) of the immortalized stem cell-derived exosome-rich conditioned medium according to the present disclosure and the ethanol fraction of the methanol extract of the rosebuds of the present disclosure (50 µg/mL) were treated together, followed by treatment with 0.1% p-dinitrophenyl-bovine serum albumin (DNP-BSA, 400 ng/mL) and cultured at 37°C for 1 hour. After obtaining the supernatant, 200 µL of 1 mM p-nitrophenyl N-acetyl-beta-D-glucosamine was added to 50 µL of the supernatant, reacted at 37°C for 3 hours, and 500 µL of sodium carbonate buffer was added to stop the enzymatic reaction. Then, the amount of β-hexosaminidase secretion was measured by absorbance at 405 nm. In this case, as a control group, a group treated with a non-immortalized stem cell normal conditioned medium was used, and as a comparison group, a group treated with an immortalized stem cell normal conditioned medium and a group treated only with an immortalized stem cell-derived exosome-rich conditioned medium of the present disclosure were used. The contents and analysis results for each control group, comparison group, and experimental group used in the experiment are shown in Table 1 below.

**[Table 1]**

| Analysis results of inhibitory efficacy on the release of β-hexosaminidase from IgE-induced RBL-2H3 cells | | | | |
|---|---|---|---|---|
| Type of stem cells | Treatment materials | Treatment amounts | IgE treatment concentration (µg/mL) | β-hexosaminidase secretion rate (%) |
| Treatment with IGE alone | - | - | 1 | 100 |
| Non-immortalized amniotic stem cells (AFSC) | Normal conditioned medium (37°C, oxygen concentration 20%) | 1% | 1 | 88.2 |
| Immortalized amniotic stem cells (CBNU-AFSC) | Normal conditioned medium (37°C, oxygen concentration 20%) | 1% | 1 | 79.1 |
| | Treatment with an exosome-rich conditioned medium of the present disclosure | 1% | 1 | 49.5 |
| | alone | | | |
| | Mixed treatment with an exosome-rich conditioned medium and a rosebud extract of the present disclosure | 1% | 1 | 32.4 |
| | | 50 µg/mL | | |
| Non-immortalized neural stem cells (NSC) | Normal conditioned medium (37°C, oxygen concentration 20%) | 1% | 1 | 75.8 |
| Immortalized neural stem cells (CBNU-NSC) | Normal conditioned medium (37°C, oxygen concentration 20%) | 1% | 1 | 67.6 |
| | Treatment with an exosome-rich conditioned medium of the present disclosure alone | 1% | 1 | 38.8 |
| | Mixed treatment with an exosome-rich conditioned medium and a rosebud extract of the present disclosure | 1% | 1 | 25.8 |
| | | 50 µg/mL | | |
| Non- | Normal conditioned | 1% | 1 | 71.2 |
| immortalized oligodendrocyte progenitor cells (NSC.olig2) | medium (37°C, oxygen concentration 20%) | | | |
| Immortalized oligodendrocyte progenitor cells (CBNU-NSC.olig2), | Normal conditioned medium (37°C, oxygen concentration 20%) | 1% | 1 | 58.7 |
| | Treatment with an exosome-rich conditioned medium of the present disclosure alone | 1% | 1 | 31.3 |
| | Mixed treatment with an exosome-rich conditioned medium and a rosebud extract of the present disclosure | 1% | 1 | 19.2 |
| | | 50 µg/mL | | |

As a result of the analysis, as for β-hexosaminidase secreted when mast cell line RBL-2H3 cells were sensitized with IgE (1 µg/mL), it was found that the secretion rate of β-hexosaminidase was decreased when treated with a conditioned medium of immortalized stem cells compared to non-immortalized stem cells. In addition, it was found that the group treated with the exosome-rich conditioned medium for each immortalized cell showed a better effect on reducing the secretion rate of β-hexosaminidase than the group treated with the normal conditioned medium. In addition, it was found that compared to the group treated with the exosome-rich conditioned medium for each immortalized cell, the group treated with the immortalized stem cell-derived exosome-rich conditioned medium of the present disclosure and the ethanol fraction of the methanol extract of rosebuds of the present disclosure exhibited the best inhibition of β-hexosaminidase secretion rate.

Specifically, the group treated with an immortalized stem cell normal conditioned medium showed a higher inhibitory effect of 28.9 to 41.3% compared to the 11.8 to 28.8% inhibition of β-hexosaminidase secretion rate in the group treated with a non-immortalized stem cell normal conditioned medium. In the case of the group treated with an immortalized stem cell-derived exosome-rich conditioned medium, the secretion inhibition rate of 50.5% in amniotic stem cells, the secretion inhibition rate of 61.2% in neural stem cells, and the secretion inhibition rate of 68.7% in oligodendrocyte progenitor cells were shown. Moreover, when the ethanol fraction of the methanol extract of rosebuds (50 µg/mL) of the present disclosure was added to the immortalized stem cell-derived exosome-rich conditioned medium, the secretion inhibitory effect of β-hexosaminidase was significantly increased, thus showing superior inhibition rate of 67.6% (immortalized amniotic stem cells), 74.2% (immortalized neural stem cells) and 80.8% (immortalized oligodendrocytes), respectively.

Accordingly, through these results, the present inventors found that the composition containing the exosome-rich conditioned medium obtained from the immortalized stem cells of the present disclosure together with the ethanol fraction of the methanol extract of rosebuds of the present disclosure has a very excellent inhibitory effect on the secretion of β-hexosaminidase, indicating that atopic dermatitis can be effectively alleviated, prevented or treated.

### <Example 4>

### Analysis of Skin Itchiness Improvement Effect according to Treatment of Immortalized Stem Cell-Derived Exosome-Rich Conditioned Medium and Rosebud Extract of Present Disclosure

The present inventors performed an experiment in the following manner to determine whether the immortalized stem cell-derived exosome-rich conditioned medium and the rosebud extract of the present disclosure exhibit a skin itchiness improvement effect.

### <4-1> Preparation of Laboratory Animals

Female ICR mice (6 weeks old) were supplied from Koatech and used after undergoing laboratory acclimatization for about 1 week. Animals were housed 5 each in cages for mice. The environment of the animal laboratory was adjusted to temperature 23 ± 2°C, relative humidity 55 ± 10%, ventilation frequency 12 times/hour, illumination cycle 12 hours (07:00-19:00), and illuminance 150 to 300 lux. Purina Rat Chow^{®}, a pellet-type solid feed for laboratory animals, was supplied from Biopia and fed. For drinking water, sterilized purified water was freely ingested. This experiment was approved by the Institutional Animal Care and Use Committee (IACUC) of the Laboratory Animal Research Center, Chungbuk National University, and was performed according to the Standard Operation Procedures (SOP) of the institution.

### <4-2> Compound 48/80-Induced Itchiness-Inducing Animal Model Production and Itchiness-Inhibiting Activity Analysis according to Treatment of Immortalized Stem Cell-Derived Exosome-Rich Conditioned Medium and Rosebud Extract of Present Disclosure

After removing the hair on the back of the mice prepared in <4-1>, 50 µL of a mixture of the immortalized stem cell-derived exosome-rich conditioned medium of the present disclosure obtained in the examples of the present disclosure and the ethanol fraction of the rosebuds (50 µg/mL) of the present disclosure was injected subcutaneously with the same amount (50 µL) of Compound-48/80 (50 µg/site) solution, respectively. The number of times of scratching with the hind paw due to itching (scratching behavior) was measured while observing the mice for 30 minutes. As a control group, a group in which a 10-fold concentrate of the non-immortalized stem cell normal conditioned medium, the immortalized stem cell normal conditioned medium, and the immortalized stem cell-derived exosome-rich conditioned medium according to the present disclosure was administered together with the Compound-48/80 (50 µg/site) solution to the mice was used.

### <4-3> Chronic Atopy Induction and Test Substance Administration

Sensitization was induced by dissolving 10 µg of ovalbumin in 200 µL of physiological saline to the mice prepared in <4-1> which were then intraperitoneally administered three times (days 0, 7, and 14) at intervals of one week. Seven days after the last sensitization (day 21), 100 µL of a mixture of the immortalized stem cell-derived exosome-rich conditioned medium of the present disclosure and the rosebud ethanol fraction (50 µg/mL) of the present disclosure and the same amount (100 µL) of ovalbumin (100 µg) was dripped onto a sterile gauze patch (1.5 × 1.5 cm) and applied to the back of each depilated mouse and fixed using a transparent dressing. The patch was replaced once every 2 days and was maintained for a total of 14 days (days 21 to 35).

### <4-4> Evaluation of Skin Lesions

For skin lesions, the patches were removed, and 24 hours later, symptoms and degrees (degree scores) were scored according to the following indicators.

### [Symptoms]

Score 0: no sign1
Score 1: erythema & hemorrhage
Score 2: edema
Score 3: excoriation & erosion
Score 4: dryness

### [Degrees]

Score 0: no sign
Score 1: mild
Score 2: moderate
Score 3: severe

The sum of the scores for each symptom measured by the above method was used as a dermatitis score for each subject.

As a result of the analysis, when Compound-48/80 (50 µg/site) was injected subcutaneously into mice, the average number of scratching was 87 times for 30 minutes, whereas when treated with non-immortalized stem cell normal conditioned medium, itching was slightly reduced by 63 to 73 times. In the group treated with the immortalized stem cell normal conditioned medium, the number of scratching was reduced by 41 to 52 times, showing a better improvement effect than the group treated with the non-immortalized stem cell normal conditioned medium. In comparison, the group treated with the immortalized stem cell-derived exosome-rich conditioned medium showed an excellent inhibitory effect such as 22 times of the immortalized amniotic stem cell-derived exosome-rich conditioned medium, 26 times of the immortalized neural stem cell-derived exosome-rich conditioned medium, and 18 times of the immortalized oligodendrocyte progenitor cell-derived exosome-rich conditioned medium. However, for this itchiness improvement effect, when treated with a mixed solution in which the ethanol fraction of the methanol extract of rosebuds of the present disclosure was added to the immortalized stem cell-derived exosome-rich conditioned medium of the present disclosure at a concentration of 50 µg/mL, the number of times of scratching in mice was 8.7 (immortalized amniotic stem cells), 11.5 (immortalized neural stem cells), and 7.1 (immortalized oligodendrocyte progenitor stem cells), respectively, confirming that itching was significantly improved (*see* FIG. 2).

In addition, the symptoms of chronic atopic dermatitis induced by ovalbumin reached 3.88 points (out of 5.00 points) as a dermatitis score index, indicating a serious level. On the other hand, atopic dermatitis symptoms were slightly improved with a score of 2.9 to 3.4 in the case of the treatment with the non-immortalized stem cell normal conditioned medium, and the group treated with the immortalized stem cell normal conditioned medium showed a better improvement effect with a score of 2.83 to 3.12. In comparison, the group treated with the immortalized stem cell-derived exosome-rich conditioned medium alone showed an excellent inhibitory effect with 1.3 points in amniotic stem cells, 1.22 points in neural stem cells, and 1.13 points in oligodendrocyte progenitor cells. When treating the mixture in which the ethanol fraction of the methanol extract of rosebuds of the present disclosure was added to the immortalized stem cell-derived exosome-rich conditioned medium of the present disclosure at a concentration of 50 µg/mL, the symptoms of chronic atopic dermatitis were significantly improved, and it was shown that the symptoms could be remarkably alleviated with a score of 0.9 in immortalized amniotic stem cells, 0.6 in immortalized neural stem cells, and 0.5 in immortalized oligodendrocyte progenitor cells (*see* FIG. 3).

### <Example 5>

### Analysis of Serum IgE and Histamine Inhibitory Effect according to Treatment of Immortalized Stem Cell-Derived Exosome-Rich Conditioned Medium and Rosebud Extract of Present Disclosure in Chronic Atopic Dermatitis Model

Blood was collected from the abdominal vena cava at the time of autopsy from the mouse experimental groups used in <Example 4>, and the collected blood was centrifuged at 3,000 rpm for 20 minutes to obtain serum. Then, total IgE in serum was measured using a mouse IgE ELISA kit, and histamine concentration was analyzed using a histamine ELISA kit.

As a result, as shown in FIG. 4, the serum IgE concentration was found to rise to 42 ng/mL according to the occurrence of chronic atopic dermatitis induced by ovalbumin, whereas in the case of treatment with an immortalized stem cell normal conditioned medium, the serum IgE concentration was slightly lowered to 34 to 38 ng/mL, whereas in the group treated with an immortalized stem cell normal conditioned medium, the serum IgE concentration was further lowered to 28.6 to 33.5 ng/mL. In addition, when the immortalized stem cell-derived exosome-rich conditioned medium was treated, there showed an excellent inhibitory effect with 11 ng/mL in the group treated with an amniotic stem cell-derived exosome-rich conditioned medium, 21 ng/mL in the group treated with a neural stem cell-derived exosome-rich conditioned medium, and 19 ng/mL in the group treated with an oligodendrocyte progenitor cell-derived exosome-rich conditioned medium. However, compared to this inhibitory effect, in the group treated by adding the ethanol fraction of the methanol extract of rosebuds to the immortalized stem cell-derived exosome-rich conditioned medium of the present disclosure at a concentration of 50 µg/mL, the inhibitory activity of serum IgE concentration was significantly increased, and the IgE concentration was significantly lowered to 2 ng/mL (immortalized amniotic stem cells), 7 ng/mL (immortalized neural stem cells) and 5 ng/mL (immortalized oligodendrocyte progenitor cells), respectively.

In addition, as shown in FIG. 5, as a result of histamine analysis in serum, it increased to 8.7 ng/mL in ovalbumin-induced atopic dermatitis, but slightly lowered to 7.1 to 8.1 ng/mL when treated with a non-immortalized stem cell normal conditioned medium, whereas an immortalized stem cell normal conditioned medium showed better inhibitory effect at 5.8 to 6.8 ng/mL. In the case of the group treated with the immortalized stem cell-derived exosome-rich conditioned medium, it showed an excellent inhibitory effect at 2.47 ng/mL in amniotic stem cells, 4.02 ng/mL in neural stem cells, and 3.97 ng/mL in oligodendrocyte progenitor cells. Compared to this inhibitory effect, when the mixture in which the ethanol fraction of the methanol extract of rosebuds of the present disclosure was added to the immortalized stem cell-derived exosome-rich conditioned medium of the present disclosure at a concentration of 50 µg/mL, the histamine inhibitory effect in serum was significantly increased, indicating that histamine concentration was significantly reduced to 1.3 ng/mL in amniotic stem cells, 1.5 ng/mL in neural stem cells, and 1.2 ng/mL in oligodendrocyte progenitor cells.

Through these results, the present inventors found that the mixture containing the immortalized stem cell-derived exosome-rich conditioned medium and the rosebud extract obtained by the method according to the present disclosure had an excellent effect of inhibiting and improving mast cell granule secretion, skin itchiness, serum IgE and histamine concentration by atopic dermatitis-inducing substances, and furthermore, had an excellent dermatitis symptom inhibitory effect, and thus can be usefully used in the manufacture of cosmetics and pharmaceuticals for the improvement of atopic dermatitis.

### <Example 6>

### Analysis of Skin Wrinkle Improvement Effect of Immortalized Stem Cell-Derived Exosome-Rich Conditioned Medium and Rosebud Extract of the Present Disclosure

The skin wrinkle improvement efficacy was evaluated as an inhibitory efficacy on MMP-1 (collagenase-1), which is one of the collagenase enzymes that decompose and disappear collagen. Bovine tendon collagen (25 mg) was dissolved in tris(hydroxymethyl)-methyl-2-aminoethane sulfonate (TES) buffer solution and allowed to stand at 37°C for 15 minutes. Then, collagenase type-1 and the sample were mixed and reacted for 5 hours. As the sample, 1% each of 10-fold concentrates of a non-immortalized stem cell normal significantly medium, an immortalized stem cell normal significantly medium, and the immortalized stem cell-derived exosome-rich conditioned medium according to the present disclosure, or a mixture in which the ethanol fraction of the methanol extract of rosebuds was added to the immortalized stem cell-derived exosome-rich conditioned medium of the present disclosure was used. After the reaction was completed, 0.2 mL of the reaction solution was taken and added to 1 mL of buffer A [4% ninhydrin in Methyl Cellosolve^{®} (2-methoxyethanol) + 0.2 M sodium citrate with 0.71 mM stannous chloride, pH 0.5], and boiled for 20 minutes. Thereafter, after adding *n*-propanol, it was allowed to stand for 15 minutes and the absorbance at 600 nm was measured to analyze the activity of the enzyme.

As a result, regarding the activity of MMP-1, an enzyme that decomposes collagen and provides the cause of skin wrinkles, as a result of treating each of the 10-fold concentrates of the stem cell conditioned medium obtained from non-immortalized amniotic stem cells (AFSC) and immortalized amniotic stem cells (CBNU-AFSC), non-immortalized neural stem cells (NSC) and immortalized neural stem cells (CBNU-NSC), non-immortalized oligodendrocyte progenitor cells (NSC.olig2) and immortalized oligodendrocyte progenitor cells (CBNU-NSC.olig2) in an amount of 1%, the group treated with the non-immortalized stem cell normal conditioned medium showed 17 to 29% MMP-1 inhibitory activity, and the immortalized stem cell normal conditioned medium showed a higher MMP-1 inhibitory activity of 37 to 47%. With respect to the group treated with the immortalized stem cell-derived exosome-rich conditioned medium, amniotic stem cells showed 67% inhibitory activity, neural stem cells showed 74% inhibitory activity, and oligodendrocyte progenitor cells showed 75% inhibitory activity. However, compared to the above results, when the mixture in which the rosebud extract (ethanol fraction of methanol extract) of the present disclosure was added to the immortalized stem cell-derived exosome-rich conditioned medium of the present disclosure at a concentration of 50 µg/mL was treated, the inhibitory effect of MMP-1 was significantly increased, indicating an excellent inhibitory effect on MMP-1 with 87% in amniotic stem cells, 89% in neural stem cells, and 84% in oligodendrocyte progenitor cells *(see* FIG. 6).

Through these results, it could be seen that when the immortalized stem cell-derived exosome-rich conditioned medium and the rosebud extract obtained by the method of the present disclosure are used together, it shows excellent inhibitory activity against MMP-1, and thus can be usefully used for skin wrinkle improvement.

### <Example 7>

### Analysis of Skin Whitening Effect of Immortalized Stem Cell-Derived Exosome-Rich Conditioned Medium and Rosebud Extract of Present Disclosure

### <7-1> Tyrosinase Inhibitory Effect

For the evaluation of the inhibitory effect on tyrosinase, an enzyme involved in the production of melanin from tyrosine, 0.1 M PBS and the corresponding sample and mushroom tyrosinase (1,500 to 2,000 U/mL) were placed in a test tube in order, and 1.5 mM tyrosine solution was added to the solution, followed by reaction at 37°C for 15 minutes. The activity of the enzyme was measured by absorbance at 490 nm using an ELISA reader. As the sample, each of 1% each of 10-fold concentrates of a non-immortalized stem cell normal conditioned medium, an immortalized stem cell normal conditioned medium, and the immortalized stem cell-derived exosome-rich conditioned medium according to the present disclosure, or a mixture (50 µg/mL) in which the ethanol fraction of the methanol extract of rosebuds was added to the immortalized stem cell-derived exosome-rich conditioned medium of the present disclosure was used.

### <7-2> Inhibitory Effect of Intracellular Melanin Production

Murine melanoma B16 F10 cells (melanocytes) were seeded in DMEM containing 10% FBS at 1 × 10⁵ cells/well, and then cultured at 37°C until the cells adhered to the bottom of the well at about 80% or more. The medium was removed and replaced with DMEM containing each sample, followed by further culture for 72 hours. The sample was the same as the sample used in <7-1>. Thereafter, the cells were recovered, the number of cells was measured, centrifuged at 10,000 rpm for 10 minutes, and the supernatant was removed and a pellet was obtained. After drying the cell pellet at 60°C, melanin was eluted with 1 M NaOH solution (100 µL) containing 10% DMSO (dimetyl sulfoxide). After measuring the absorbance at 490 nm with a microplate reader, the amount of melanin per cell number was calculated.

As a result of analyzing the activity of tyrosinase, an enzyme that synthesizes melanin from tyrosine and provides the cause of pigmentation, in the case of treating each of the 10-fold concentrates of the stem cell conditioned media obtained from non-immortalized amniotic stem cells (AFSC) and immortalized amniotic stem cells (CBNU-AFSC), non-immortalized neural stem cells (NSC) and immortalized neural stem cells (CBNU-NSC), non-immortalized oligodendrocyte progenitor cells (NSC.olig2) and immortalized oligodendrocyte progenitor cells (CBNU-NSC.olig2) in an amount of 1%, the group treated with the non-immortalized stem cell normal conditioned medium showed 9 to 32% tyrosinase inhibitory activity, and the group treated with the immortalized stem cell normal conditioned medium showed a higher tyrosinase inhibitory activity of 25 to 56.5%. With respect to the group treated with the immortalized stem cell-derived exosome-rich conditioned medium, 45% inhibitory activity showed in amniotic stem cells, 79% inhibitory activity showed in neural stem cells, and 73% inhibitory activity showed in oligodendrocyte progenitor cells. However, in comparison, in the group treated by adding the ethanol fraction of the methanol extract of rosebuds of the present disclosure to the immortalized stem cell-derived exosome-rich conditioned medium according to the present disclosure at a concentration of 50 µg/mL, the tyrosinase inhibitory activity was significantly increased, indicating an excellent inhibitory effect with 79% in amniotic stem cells, 92% in neural stem cells, and 87% in oligodendrocyte progenitor cells (*see* FIG. 7).

In addition, as a result of analyzing the inhibitory efficacy on direct melanin production in melanocytes, the group treated with the non-immortalized stem cell normal conditioned medium showed an inhibitory effect of 13 to 34%, whereas the group treated with the immortalized stem cell normal conditioned medium showed a higher inhibitory effect of 35 to 51%. In comparison, the immortalized stem cell-derived exosome-rich conditioned medium showed excellent inhibitory effects of 54% in amniotic stem cells, 69% in neural stem cells, and 72% in oligodendrocyte progenitor cells. In particular, in the group treated by adding the ethanol fraction of the methanol extract of rosebuds of the present disclosure to the immortalized stem cell-derived exosome-rich conditioned medium of the present disclosure at a concentration of 50 µg/mL, the melanin production inhibitory effect was significantly increased, indicating an excellent melanin inhibitory effect in amniotic stem cells by 83%, neural stem cells by 89%, and oligodendrocyte progenitor cells by 94%, respectively (*see* FIG. 8).

Accordingly, through these results, the present inventors found that when the immortalized stem cell-derived exosome-rich conditioned medium and the rosebud extract obtained by the method according to the present disclosure are used together, the activity of tyrosinase and melanin production from melanocytes may be very remarkably inhibited simultaneously, and thus may be usefully used in the manufacture of cosmetics for skin whitening.

Hereinbefore, the present disclosure has been described with reference to the preferred embodiments. The scope of the present disclosure is defined by the appended claims.

### [Accession No.]

Name of Depository Institution: Korea Research Institute of Bioscience & Biotechnology
Address of Depository Institution: (Shinjung-dong) 181 Ipsin-gil, Jeongeup-si, Jeollabuk-do, Korea [56212]
Accession No.: KCTC12634BP
Deposit Date: 20140725
Name of Depository Institution: Korea Research Institute of Bioscience & Biotechnology
Address of Depository Institution: (Shinjung-dong) 181 Ipsin-gil, Jeongeup-si, Jeollabuk-do, Korea [56212]
Accession No.: KCTC12635BP
Deposit Date: 20140725
Name of Depository Institution: Korea Research Institute of Bioscience & Biotechnology
Address of Depository Institution: (Shinjung-dong) 181 Ipsin-gil, Jeongeup-si, Jeollabuk-do, Korea [56212]
Accession No.: KCTC12636BP
Deposit Date: 20140725

### [Industrial Applicability]

The composition containing the immortalized stem cell-derived exosome-rich conditioned medium and the rosebud extract as active ingredients, according to the present disclosure, has excellent activity in inhibiting mast cell granule secretion, inhibiting immunoglobulin (IgE) in serum, inhibiting histamines in serum, and improving itchiness, is excellent in tyrosinase inhibitory activity and melanin production inhibitory activity, and may maximize the activity of active ingredients by overcoming the skin penetration issue of stem cell-derived ingredients, and thus may be usefully used in the manufacture of a cosmetic composition having activity in atopic dermatitis improvement, skin wrinkle improvement, and whitening as well as may also be usefully used in the manufacture of a pharmaceutical composition for the prevention or treatment of atopic dermatitis and pigmentation diseases.

## Claims

1. A composition comprising an immortalized stem cell-derived exosome-rich conditioned medium and a rosebud extract, wherein the rosebud extract is a solvent fraction obtained by extraction and fractionation by adding any one of solvents selected from the group consisting of ethanol, butanol and ethyl acetate to a methanol ultrasonic extract obtained by adding methanol to dried and pulverized rosebuds and sonicating the same.

2. The composition according to claim 1, wherein the immortalized stem cells are immortalized amniotic stem cells, immortalized neural stem cells, or immortalized oligodendrocyte progenitor cells.

3. The composition according to claim 2, wherein the immortalized amniotic stem cells are immortalized amniotic stem cells, CBNU-AFSC cell line (Accession No.: KCTC12634BP), the immortalized neural stem cells are immortalized neural stem cells, CBNU-NSC cell line (Accession No.: KCTC12635BP), and the immortalized oligodendrocyte progenitor cells are immortalized oligodendrocyte progenitor cells, CBNU-NSC.olig2 cell line (Accession No.: KCTC12636BP).

4. The composition according to claim 1, wherein the immortalized stem cell-derived exosome-rich conditioned medium is obtained by treating with TNF-α and interferon gamma (IFN-y) in a culture medium containing immortalized stem cells, and culturing the same under a low oxygen concentration condition of 1 to 5% for 12 to 72 hours.

5. The composition according to claim 4, wherein the immortalized stem cell-derived conditioned medium is treated with TNF-α at a concentration of 3 to 100 ng/ml, and with interferon gamma (IFN-y) at a concentration of 1 to 10 U/ml.

6. The composition according to claim 1, wherein 80% methanol is used for extraction followed by extraction with 70% ethanol, or 70% butanol, or 70% ethyl acetate, preferably with extraction with 70% ethanol.

7. The composition according to claim 1, wherein the rosebud is a rosebud of a rose selected from the group consisting of Icewing *(Rosa hybrida* Icewing), Onnuri (*Rosa hybrida* Onnuri), Colorado *(Rosa hybrida* Colorado) and rose vines (*Rosa multiflora* Platyphylla thory).

8. The composition according to claim 1, wherein the composition has activity in atopic dermatitis improvement, skin wrinkle improvement or whitening.

9. A method for preparing a composition having activity in atopic dermatitis improvement, skin wrinkle improvement and whitening, the method comprising:
(1) simultaneously treating a culture medium containing immortalized stem cells with TNF-α and interferon gamma (IFN-y), and culturing the same under a low oxygen concentration condition of 1 to 5% for 12 to 72 hours to obtain an immortalized stem cell-derived exosome-rich conditioned medium;
(2) adding any one of solvents selected from the group consisting of ethanol, butanol and ethyl acetate to a methanol ultrasonic extract obtained by adding methanol to dried and pulverized rosebuds and sonicating the same, followed by extraction and fractionation to obtain a solvent fraction for the methanol extract of the rosebuds; and
(3) mixing the immortalized stem cell-derived exosome-rich conditioned medium obtained in the process (1) and the solvent fraction for the methanol extract of the rosebuds obtained in the process (2).

10. The method according to claim 9, wherein the immortalized stem cells are immortalized amniotic stem cells, CBNU-AFSC cell line (Accession No.: KCTC12634BP), the immortalized neural stem cells are immortalized neural stem cells, CBNU-NSC cell line (Accession No.: KCTC12635BP), and the immortalized oligodendrocyte progenitor cells are immortalized oligodendrocyte progenitor cells, CBNU-NSC.olig2 cell line (Accession No.: KCTC12636BP).

11. The method according to claim 9, wherein in the process (1), the immortalized stem cell-derived exosome-rich conditioned medium is treated with TNF-α at a concentration of 3 to 100 ng/ml, and with interferon gamma (IFN-y) at a concentration of 1 to 10 U/ml.

12. A composition comprising an immortalized stem cell-derived exosome-rich conditioned medium and a rosebud extract for use in a method for the preventing or treating atopic dermatitis or pigmentation diseases.

13. A composition for use in a method for the preventing or treating atopic dermatitis or pigmentation diseases according to claim 12 comprising an immortalized stem cell-derived exosome-rich conditioned medium and a rosebud extract, wherein the pigmentation disease is one or more of freckles, senile spots, chloasma, small brown spots, brown or black moles, sunshine pigment spots, cyanic melisma, hyperpigmentation after drug use, adverse sequelae following tissue sclerotherapy, gravidic chloasma, melasma in women taking oral contraceptives, hyperpigmentation after inflammation caused by lesions or skin inflammation like excoriation and burn, phototoxic reactions, or other similar small, fixed pigmented lesions.

## Patentansprüche

1. Zusammensetzung, umfassend ein aus immortalisierten Stammzellen gewonnenes, exosomreiches, konditioniertes Medium und einen Rosenknospenextrakt, wobei der Rosenknospenextrakt eine Lösungsmittelfraktion ist, erhalten durch Extraktion und Fraktionierung durch Zugabe eines beliebigen Lösungsmittels ausgewählt aus der Gruppe bestehend aus Ethanol, Butanol und Ethylacetat zu einem Methanol-Ultraschallextrakt, der durch Zugabe von Methanol zu getrockneten und pulverisierten Rosenknospen und deren Beschallung erhalten wird.

2. Zusammensetzung gemäß Anspruch 1, wobei die immortalisierten Stammzellen immortalisierte Amnionstammzellen, immortalisierte neuronale Stammzellen oder immortalisierte Oligodendrozyten-Vorläuferzellen sind.

3. Zusammensetzung gemäß Anspruch 2, wobei die immortalisierten Amnionstammzellen immortalisierte Amnionstammzellen, CBNU-AFSC-Zelllinie (Zugangsnummer: KCTC12634BP) sind, die immortalisierten neuronalen Stammzellen immortalisierte neuronale Stammzellen, CBNU-NSC-Zelllinie (Zugangsnummer: KCTC12635BP) sind und die immortalisierten Oligodendrozyten-Vorläuferzellen immortalisierte Oligodendrozyten-Vorläuferzellen, CBNU-NSC.olig2-Zelllinie (Zugangsnummer: KCTC12636BP) sind.

4. Zusammensetzung gemäß Anspruch 1, wobei das aus immortalisierten Stammzellen gewonnene exosomreiche konditionierte Medium erhalten wird durch Behandlung mit TNF-α und Interferon-gamma (IFN-γ) in einem Kulturmedium, das immortalisierte Stammzellen enthält, und Kultivierung desselben unter einer Bedingung mit niedriger Sauerstoffkonzentration von 1 bis 5 % für 12 bis 72 Stunden.

5. Zusammensetzung gemäß Anspruch 4, wobei das aus immortalisierten Stammzellen gewonnene konditionierte Medium mit TNF-α in einer Konzentration von 3 bis 100 ng/ml und mit Interferon-gamma (IFN-γ) in einer Konzentration von 1 bis 10 U/ml behandelt wird.

6. Zusammensetzung gemäß Anspruch 1, wobei 80 % Methanol zur Extraktion verwendet wird, gefolgt von einer Extraktion mit 70 % Ethanol oder 70 % Butanol oder 70 % Ethylacetat, vorzugsweise mit einer Extraktion mit 70 % Ethanol.

7. Zusammensetzung gemäß Anspruch 1, wobei die Rosenknospe eine Rosenknospe einer Rose ist ausgewählt aus der Gruppe bestehend aus Icewing *(Rosa hybrida* Icewing), Onnuri *(Rosa hybrida* Onnuri), Colorado *(Rosa hybrida* Colorado) und Rosenreben (*Rosa multiflora* Platyphylla thory).

8. Zusammensetzung gemäß Anspruch 1, wobei die Zusammensetzung eine Wirkung bei der Verbesserung von atopischer Dermatitis, bei der Verbesserung von Hautfalten oder bei der Hautaufhellung aufweist.

9. Verfahren zur Herstellung einer Zusammensetzung mit Wirkung zur Verbesserung von atopischer Dermatitis, zur Verbesserung von Hautfalten und zur Hautaufhellung, wobei das Verfahren umfasst:
(1) gleichzeitiges Behandeln eines Kulturmediums, das immortalisierte Stammzellen enthält, mit TNF-α und Interferon-gamma (IFN-γ) und Kultivieren desselben unter einer Bedingung mit niedriger Sauerstoffkonzentration von 1 bis 5 % für 12 bis 72 Stunden, um ein aus immortalisierten Stammzellen gewonnenes exosomenreiches konditioniertes Medium zu erhalten;
(2) Zugeben eines beliebigen Lösungsmittels ausgewählt aus der Gruppe bestehend aus Ethanol, Butanol und Ethylacetat zu einem Methanol-Ultraschallextrakt, erhalten durch Zugabe von Methanol zu getrockneten und pulverisierten Rosenknospen und Ultraschallbehandlung derselben, gefolgt von Extraktion und Fraktionierung, um eine Lösungsmittel-Fraktion für den Methanolextrakt der Rosenknospen zu erhalten; und
(3) Mischen des im Verfahrensschritt (1) erhaltenen, aus immortalisierten Stammzellen gewonnenen, exosomreichen konditionierten Mediums und der Lösungsmittelfraktion für den Methanolextrakt der Rosenknospen, die im Verfahren (2) erhalten wurden.

10. Verfahren nach Anspruch 9, wobei die immortalisierten Stammzellen immortalisierte Amnionstammzellen, CBNU-AFSC-Zelllinie (Zugangsnummer: KCTC12634BP) sind, die immortalisierten neuronalen Stammzellen immortalisierte neurale Stammzellen, CBNU-NSC-Zelllinie (Zugangsnummer: KCTC12635BP) und die immortalisierten Oligodendrozyten-Vorläuferzellen immortalisierte Oligodendrozyten-Vorläuferzellen der CBNU-NSC.olig2-Zelllinie (Zugangsnummer: KCTC12636BP) sind.

11. Verfahren nach Anspruch 9, wobei in Verfahrensschritt (1) das aus immortalisierten Stammzellen gewonnene, exosomreiche, konditionierte Medium mit TNF-α in einer Konzentration von 3 bis 100 ng/ml und mit Interferon-gamma (IFN-γ) in einer Konzentration von 1 bis 10 U/ml behandelt wird.

12. Zusammensetzung, umfassend ein aus immortalisierten Stammzellen gewonnenes, exosomreiches, konditioniertes Medium und einen Rosenknospenextrakt zur Verwendung in einem Verfahren zur Vorbeugung oder Behandlung von atopischer Dermatitis oder Pigmentstörungen.

13. Zusammensetzung zur Verwendung in einem Verfahren zur Vorbeugung oder Behandlung von atopischer Dermatitis oder Pigmentstörungen gemäß Anspruch 12, umfassend ein aus immortalisierten Stammzellen gewonnenes, exosomreiches Konditionierungsmedium und einen Rosenknospenextrakt, wobei die Pigmentstörung eine oder mehrere der folgenden ist: Sommersprossen, Altersflecken, Chloasma, kleine braune Flecken, braune oder schwarze Muttermale, Sonnenpigmentflecken, cyanisches Melasma, Hyperpigmentierung nach Medikamenteneinnahme, unerwünschte Folgeerscheinungen nach Gewebeverödung, gravidäres Chloasma, Melasma bei Frauen, die orale Kontrazeptiva einnehmen, Hyperpigmentierung nach Entzündungen durch Läsionen oder Hautentzündungen wie Exkoriationen und Verbrennungen, phototoxische Reaktionen oder andere ähnliche kleine, fixierte pigmentierte Läsionen.

## Revendications

1. Composition comprenant un milieu conditionné riche en exosomes, dérivé de cellules souches immortalisées et un extrait de bouton de rose, dans laquelle ledit extrait de bouton de rose est une fraction de solvant obtenue par extraction et fractionnement par addition d'un solvant quelconque choisi dans le groupe constitué par l'éthanol, le butanol et l'acétate d'éthyle, à un extrait ultrasonique de méthanol obtenu par addition de méthanol à des boutons de rose séchés et pulvérisés, et la sonication de ceux-ci.

2. Composition selon la revendication 1, dans laquelle les cellules souches immortalisées sont des cellules souches amniotiques immortalisées, des cellules souches neurales immortalisées ou des cellules progénitrices d'oligodendrocytes immortalisées.

3. Composition selon la revendication 2, dans laquelle les cellules souches amniotiques immortalisées sont des cellules souches amniotiques immortalisées, lignée cellulaire CBNU-AFSC (n° d'accès : KCTC12634BP), les cellules souches neurales immortalisées sont des cellules souches neurales immortalisées, lignée cellulaire CBNU-NSC (n° d'accès : KCTC12635BP), et les cellules progénitrices d'oligodendrocytes immortalisées sont des cellules progénitrices d'oligodendrocytes immortalisées, lignée cellulaire CBNU-NSC.olig2 (n° d'accès : KCTC12636BP).

4. Composition selon la revendication 1, dans laquelle le milieu conditionné riche en exosomes, dérivé de cellules souches immortalisées est obtenu par traitement avec du TNF-α et de l'interféron gamma (IFN-y) dans un milieu de culture contenant des cellules souches immortalisées, et par culture de ce dernier dans des conditions de faible concentration en oxygène de 1 à 5 %, pendant 12 à 72 heures.

5. Composition selon la revendication 4, dans laquelle le milieu conditionné dérivé de cellules souches immortalisées est traité avec du TNF-α à une concentration de 3 à 100 ng/ml et avec de l'interféron gamma (IFN-y) à une concentration de 1 à 10 U/ml.

6. Composition selon la revendication 1, dans laquelle 80 % de méthanol sont utilisés pour l'extraction, suivie d'une extraction à 70 % d'éthanol ou à 70 % de butanol ou à 70 % d'acétate d'éthyle, de préférence avec une extraction à 70 % d'éthanol.

7. Composition selon la revendication 1, dans laquelle le bouton de rose est un bouton de rose d'une rose choisie dans le groupe constitué par Icewing (*Rosa hybrida* Icewing), Onnuri (*Rosa hybrida* Onnuri), Colorado (*Rosa hybrida* Colorado) et des vignes de rose (*Rosa multiflora* Platyphylla thory).

8. Composition selon la revendication 1, dans laquelle la composition présente une activité dans l'amélioration de la dermatite atopique, l'atténuation des rides ou le blanchiment de la peau.

9. Procédé de préparation d'une composition ayant de l'activité dans l'amélioration de la dermatite atopique, l'atténuation des rides et le blanchiment de la peau, le procédé comprenant :
(1) le traitement simultané d'un milieu de culture contenant des cellules souches immortalisées avec du TNF-α et de l'interféron gamma (IFN-γ), et la culture de celui-ci dans des conditions de faible concentration en oxygène de 1 à 5 %, pendant 12 à 72 heures, afin d'obtenir un milieu conditionné riche en exosomes, dérivé de cellules souches immortalisées ;
(2) l'ajout d'un solvant quelconque choisi dans le groupe constitué par l'éthanol, le butanol et l'acétate d'éthyle, à un extrait ultrasonique de méthanol obtenu par addition de méthanol à des boutons de rose séchés et pulvérisés, et la sonication de ceux-ci, puis l'extraction et le fractionnement pour obtenir une fraction de solvant pour l'extrait de méthanol des boutons de rose ; et
(3) mélanger le milieu conditionné riche en exosomes, dérivé de cellules souches immortalisées, obtenu selon l'étape de procédé (1), et la fraction de solvant pour l'extrait de méthanol des boutons de rose obtenu selon le procédé (2).

10. Procédé selon la revendication 9, dans lequel les cellules souches immortalisées sont des cellules souches amniotiques immortalisées, lignée cellulaire CBNU-AFSC (n° d'accès : KCTC12634BP), les cellules souches neurales immortalisées sont des cellules souches neurales immortalisées, lignée cellulaire CBNU-NSC (n° d'accès : KCTC12635BP), et les cellules progénitrices d'oligodendrocytes immortalisées sont des cellules progénitrices d'oligodendrocytes immortalisées de la lignée cellulaire CBNU-NSC.olig2 (n° d'accès : KCTC12636BP).

11. Procédé selon la revendication 9, dans lequel, dans l'étape de procédé (1), le milieu conditionné riche en exosomes, dérivé de cellules souches immortalisées est traité avec du TNF-α à une concentration de 3 à 100 ng/ml, et avec de l'interféron gamma (IFN-y) à une concentration de 1 à 10 U/ml.

12. Composition comprenant un milieu conditionné riche en exosomes, dérivé de cellules souches immortalisées et un extrait de bouton de rose, destinée à être utilisée dans un procédé de prévention ou de traitement de la dermatite atopique ou des maladies pigmentaires.

13. Composition destinée à être utilisée dans un procédé de prévention ou de traitement de la dermatite atopique ou des maladies pigmentaires selon la revendication 12, comprenant un milieu conditionné riche en exosomes, dérivé de cellules souches immortalisées et un extrait de bouton de rose, dans laquelle la maladie pigmentaire est une ou plusieurs des affections suivantes : taches de rousseur, taches de vieillesse, chloasma, petites taches brunes, grains de beauté bruns ou noirs, taches pigmentaires solaires, mélisme cyanique, hyperpigmentation après usage médicamenteux, séquelles indésirables suites à une sclérothérapie tissulaire, chloasma gravidique, mélasma chez les femmes sous contraceptifs oraux, hyperpigmentation après inflammation causée par des lésions ou une inflammation cutanée telle qu'une excoriation ou une brûlure, réactions phototoxiques ou autres petites lésions pigmentaires fixes similaires.
